# EUROPÄISCHE PATENTANMELDUNG

(11) **EP 2 194 080 A1**
(43) Veröffentlichungstag der Anmeldung: **09.06.2010**
(21) Anmeldenummer: 10000385.4
(22) Anmeldetag: 28.11.2001
(51) Int. Cl.: C08F 291/00, C08F 290/06, C08F 2/00

(54) **Tensidhaltige kosmetische, dermatologische und pharmazeutische Mittel**

(30) Priorität: 01.12.2000 DE 10059819
(62) Teilanmeldung aus: 01998592.8
(71) Anmelder: Clariant Produkte (Deutschland) GmbH, 65929 Frankfurt am Main (DE)
(72) Erfinder: Loeffler, Matthias, 65510 Idstein (DE); Morschhaeuser, Roman, 55122 Mainz (DE); Kayser, Christoph, 55127 Mainz (DE)
(74) Vertreter: Paczkowski, Marcus

(57) **Zusammenfassung**

Gegenstand der Erfindung sind tensidhaltige kosmetische, dermatologische und pharmazeutische Mittel, enthaltend mindestens ein wasserlösliches oder wasserquellbares Copolymer, erhältlich durch radikalische Copolymerisation von
A) Acryloyldimethyltaurinsäure und/oder Acryloyldimethyltauraten,
B) gegebenenfalls einem oder mehreren weiteren olefinisch ungesättigten, nicht kationischen, Comonomeren,
C) gegebenenfalls einem oder mehreren olefinisch ungesättigten, kationischen Comonomeren,
D) gegebenenfalls einer siliziumhaltigen Komponente(n),
E) gegebenenfalls einer fluorhaltigen Komponente(n),
F) gegebenenfalls einem oder mehreren Makromonomeren,
G) wobei die Copolymerisation gegebenenfalls in Gegenwart mindestens eines polymeren Additivs erfolgt,
H) mit der Maßgabe, dass die Komponente A) mit mindestens einer Komponente ausgewählt aus einer der Gruppen D) bis G) copolymerisiert wird.

## Beschreibung

Die vorliegende Erfindung betrifft tensidhaltige kosmetische, dermatologische und pharmazeutische Mittel, enthaltend kammförmige Copolymere auf Basis von Acryloyldimethyltaurinsäure.

Auf dem Gebiet der Haut- und Haarkosmetik hat in den letzten Jahren eine bis heute nicht abgeschlossene stürmische Entwicklung eingesetzt. Immer neue, verbesserte und dem aktuellen Trend entsprechende Produkte werden den Verbrauchern angeboten. Ein Schwerpunkt ist es, Rinse Off Formulierungen zu entwickeln, die nicht nur eine effektive Reinigung von Haut oder Haar gewährleisten, sondern zusätzliche vorteilhafte Eigenschaften vermitteln. So ist man z.B. bemüht, in Shampooformulierungen Eigenschaften wie Sensorik, Konditionierung, Glanz, Nass- und Trockenkämmbarkeit und Farbtiefe zu verbessern. Ferner sollten die Shampooformulierungen durch kürzere Trocknungszeiten die Hitzebelastung der Haare beim Fönen verringern und bereits vorhandene Haarschädigungen, wie z.B. Spliss, "reparieren". Solch eine konditionierende Shampooformulierung wäre vorteilhaft, da der Kauf und die Verwendung von separatem Shampoo und Haarnachbehandlungsmittel entfallen würde. Die Patentliteratur enthält zahlreiche Vorschläge zur Realisierung eines solchen Vorhabens, darunter die Verwendung von nichtionischen und amphoteren Tensiden, wasserlöslichen Polymeren, kationischen Fettsäurederivaten, wasserlöslichen Silikonen und Emulsionen von Silikonen und anderen Ölen. Jedoch haben sich bis heute nur wenige zufriedenstellenden Lösungen ergeben. Ein Problem bei der Verwendung von kationischen Fettsäurederivaten besteht in der Unverträglichkeit mit gut reinigenden anionischen Tensiden. Ferner erbringen kationische Konditionierer nicht das geforderte Maß an Weichheit.

Häufig benutzte Materialien, die das Haar geschmeidig und weich erscheinen lassen, sind Silikonderivate. Lösliche oder kationische Silikone haben jedoch wesentliche Nachteile, da die löslichen Silikonderivate ungenügende Konditioniereigenschaften (mangelhafte Ablagerung auf das Haar aus der Lösung heraus), haben während die kationischen Typen wiederum nicht mit den anionischen Tensiden verträglich sind und unwirksame Komplexe bilden.

Unlösliche Silikone, wie sie von H. C. Green in US-Patent A-2,826,551 vorgeschlagen wurden, lassen sich oft nur unzureichend in Shampooformulierungen einarbeiten. Das Problem besteht hier in der Erzeugung einer Suspension der feinverteilten, unlöslichen Polymere, die über einen längeren Zeitraum stabil sein soll. Eine Vielzahl von Verbindungen wurden den silikonhaltigen Formulierungen zugesetzt, um eine Verdickung und Stabilisierung zu bewirken. Die bisher erfolgreichste Vorgehensweise wird in EP 0,181,773 offenbart, wo die Verwendung langkettiger Acylderivate zu der Bildung von stabilen Formulierungen enthaltend unlösliche Silikone führte. Die beschriebenen Acylderivate nach dem Stand der Technik enthalten Fettsäurealkanolamide, Fettsäuredialkanolamide, Alkanolamide und deren Derivate. Diese Amide stehen jedoch im Verdacht, an der Bildung von Nitrosaminen beteiligt zu sein. Es ist daher erwünscht, kosmetische Zubereitungen ohne solche Derivate zu formulieren.

Das flüssige Tensidsystem muss zudem auch eine Viskosität haben, die dem jeweiligen Verwendungszweck angepasst ist und sich möglichst variabel einstellen lässt. So ist die Viskosität ein entscheidendes Kriterium für die Qualität eines flüssigen Tensidpräparates. Es werden z.B. von einem Duschgel relativ hohe Viskositäten gefordert, während ein Haarshampoo gewöhnlich eine fließfähige Flüssigkeit mit einer relativ niedrigen Viskosität darstellt.

Verdickung von Tensidsystemen kann im einfachsten Fall durch Zugabe von Elektrolyten erfolgen, aber auch Cellulose Derivate, natürliche Polymere (z.B. Xanthan Gum, Guar), nichtionische Tenside oder Polyethylenglykol-Derivate finden Einsatz.

Alle diese Verdickersysteme führen zu Tensidsystemen, die strukturviskos und/oder thixotrop sind. Je nach Verdicker sind allerdings auch diverse Nachteile in Kauf zu nehmen:
Elektrolyte sind korrosiv, zudem ist die Verdickerleistung stark abhängig vom jeweiligen Tensidsystem (viele Tenside können durch Elektrolytzugabe nicht verdickt werden).
Die Verdickungsleistung von Cellulose Derivaten wird durch Salz stark herabgesetzt. Viele Tenside, z.B. Betain, enthalten hohe Mengen an Salz, was die Verwendung von Cellulose Derivaten einschränkt..
Natürliche Polymere sind sehr schwierig zu verarbeiten, zudem sind klare Formulierungen häufig nicht realisierbar.
Bekannte nichtionische Verdickungsmittel für flüssige Tensidformulierungen sind unter anderem Fettsäurealkanolamide. Als Fettsäurealkanolamid wird in der Praxis bevorzugt das Kokosfettsäurediethanolamid eingesetzt. Es zeigt gegenüber anderen Fettsäurediethanolamiden die besten Verdickungseigenschaften. Nachteilig ist jedoch das Vorhandensein von aminischen Nebenverbindungen/Verunreinigungen.
Polyethylenglykol-Derivate wie PEG 6000 Distearate, PEG-120 Methyl Glucose Dioleate, PEG-150 Pentaerythrityl Tetrastearate, PEG-20 Methyl Glucose Sesquistearate etc, sind sowohl aufwendig in der Herstellung (mangelnde Konformität von batch zu batch), als auch teilweise kompliziert zu verarbeiten (Aufschmelzen bzw. Lösen bei hoher Temperatur, hohe Einsatzmengen).

Überraschend wurde nun gefunden, dass sich eine neue Klasse von Kammpolymeren auf Basis von Acryloyldimethyltaurinsäure (AMPS)- die sich sowohl als Verdicker, Konsistenzgeber, Emulgator, Dispergator, Gleitmittel, Conditioner und/oder Stabilisator eignet- hervorragend zur Formulierung von tensidhaltigen kosmetischen, dermatologischen und pharmazeutischen Mitteln eignet.

Gegenstand der Erfindung sind tensidhaltige kosmetische, dermatologische und pharmazeutische Mittel, enthaltend mindestens ein wasserlösliches oder wasserquellbares Copolymer, erhältlich durch radikalische Copolymerisation von
A) Acryloyldimethyltaurinsäure und/oder Acryloyldimethyltauraten,
B) gegebenenfalls einem oder mehreren weiteren olefinisch ungesättigten, nicht kationischen, gegebenenfalls vernetzenden, Comonomeren, die wenigstens ein Sauerstoff-, Stickstoff-, Schwefel- oder Phosphoratom aufweisen und ein Molekulargewicht kleiner 500 g/mol besitzen,
C) gegebenenfalls einem oder mehreren olefinisch ungesättigten, kationischen Comonomeren, die wenigstens ein Sauerstoff-, Stickstoff-, Schwefel- oder Phosphoratom aufweisen und ein Molekulargewicht kleiner 500 g/mol besitzen,
D) gegebenenfalls einer oder mehreren mindestens monofunktionellen, zur radikalischen Polymerisation befähigten, siliziumhaltigen Komponente(n),
E) gegebenenfalls einer oder mehreren mindestens monofunktionellen, zur radikalischen Polymerisation befähigten, fluorhaltigen Komponente(n),
F) gegebenenfalls einem oder mehreren einfach oder mehrfach olefinisch ungesättigten, gegebenenfalls vernetzenden, Makromonomeren, die jeweils mindestens ein Sauerstoff-, Stickstoff-, Schwefel- oder Phosphoratom besitzen und ein zahlenmittleres Molekulargewicht größer oder gleich 200 g/mol aufweisen, wobei es sich bei den Makromonomeren nicht um eine siliziumhaltige Komponente D) oder fluorhaltige Komponente E) handelt,
G) wobei die Copolymerisation gegebenenfalls in Gegenwart mindestens eines polymeren Additivs mit zahlenmittleren Molekulargewichten von 200 g/mol bis 10⁹ g/mol erfolgt,
H) mit der Maßgabe, dass die Komponente A) mit mindestens einer Komponente ausgewählt aus einer der Gruppen D) bis G) copolymerisiert wird.

Die erfindungsgemäßen Copolymere besitzen bevorzugt ein Molekulargewicht von 10³ g/mol bis 10⁹ g/mol, besonders bevorzugt von 10⁴ bis 10⁷ g/mol, insbesondere bevorzugt 5*10⁴ bis 5*10⁶ g/mol.

Bei den Acryloyldimethyltauraten kann es sich um die anorganischen oder organischen Salze der Acryloyldimethyltaurinsäure (Acrylamidopropyl-2-methyl-2-sulfonsäure) handeln. Bevorzugt werden die Li⁺-, Na⁺-, K⁺-, Mg⁺⁺-, Ca⁺⁺-, Al⁺⁺⁺-und/oder NH₄⁺-Salze. Ebenfalls bevorzugt sind die Monoalkylammonium-, Dialkylammonium-, Trialkylammonium- und/oder Tetraalkylammoniumsalze, wobei es sich bei den Alkylsubstituenten der Amine unabhängig voneinander um (C₁-C₂₂)-Alkylreste oder (C₂-C₁₀)-Hydroxyalkylreste handeln kann. Weiterhin sind auch ein bis dreifach ethoxylierte Ammoniumverbindungen mit unterschiedlichem Ethoxylierungsgrad bevorzugt. Es sollte angemerkt werden, dass auch Mischungen von zwei- oder mehreren der oben genannten Vertreterim Sinne der Erfindung sind.

Der Neutralisationsgrad der Acryloyldimethyltaurinsäure kann zwischen 0 und 100 % betragen, besonders bevorzugt ist ein Neutralisationsgrad von oberhalb 80%.

Bezogen auf die Gesamtmasse der Copolymere beträgt der Gehalt an Acryloyldimethyltaurinsäure bzw. Acryloyldimethyltauraten mindestens 0,1 Gew.-%, bevorzugt 20 bis 99,5 Gew.-%, besonders bevorzugt 50 bis 98 Gew.-%.

Als Comonomere B) können alle olefinisch ungesättigten, nicht kationischen Monomere eingesetzt werden, deren Reaktionsparameter eine Copolymerisation mit Acryloyldimethyltaurinsäure und/oder Acryloyldimethyltauraten in den jeweiligen Reaktionsmedien erlauben.
Als Comonomere B) bevorzugt sind ungesättigte Carbonsäuren und deren Anhydride und Salze, sowie deren Ester mit aliphatischen, olefinischen, cycloaliphatischen, arylaliphatischen oder aromatischen Alkoholen mit einer Kohlenstoffzahl von 1 bis 30.
Als ungesättigte Carbonsäuren besonders bevorzugt sind Acrylsäure, Methacrylsäure, Styrolsulfonsäure, Maleinsäure, Fumarsäure, Crotonsäure, Itaconsäure und Seneciosäure.
Als Gegenionen bevorzugt sind Li⁺, Na⁺, K⁺, Mg⁺⁺, Ca⁺⁺, Al⁺⁺⁺, NH₄⁺, Monoalkylammonium-, Dialkylammonium-, Trialkylammonium- und/oder Tetraalkylammoniumreste, wobei es sich bei den Alkylsubstituenten der Amine unabhängig voneinander um (C₁-C₂₂)-Alkylreste oder (C₂-C₁₀)-Hydroxyalkylreste handeln kann. Zusätzlich können auch ein bis dreifach ethoxylierte Ammoniumverbindungen mit unterschiedlichem Ethoxylierungsgrad Anwendung finden. Der Neutralisationsgrad der Carbonsäuren kann zwischen 0 und 100% betragen.
Als Comonomere B) weiterhin bevorzugt sind offenkettige N-Vinylamide, bevorzugt N-Vinylformamid (VIFA), N-Vinylmethylformamid,
N-Vinylmethylacetamid (VIMA) und N-Vinylacetamid; cyclische N-Vinylamide (N-Vinyllactame) mit einer Ringgröße von 3 bis 9, bevorzugt N-Vinylpyrrolidon (NVP) und N-Vinylcaprolactam; Amide der Acryl- und Methacrylsäure, bevorzugt Acrylamid, Methacrylamid, N,N-Dimethyl-acrylamid, N,N-Diethylacrylamid und N,N-Diisopropylacrylamid; alkoxylierte Acryl- und Methacrylamide, bevorzugt Hydroxyethylmethacrylat, Hydroxymethylmethacrylamid, Hydroxyethylmethacrylamid, Hydroxypropylmethacrylamid und
Bernsteinsäuremono-[2-(methacryloyloxy)-ethylester];
N,N-Dimethylaminomethacrylat; Diethylamino-methylmethacrylat; Acryl- und
Methacrylamidoglykolsäure; 2- und 4-Vinylpyridin; Vinylacetat;
Methacrylsäureglycidylester; Styrol; Acrylnitril; Vinylchlorid; Stearylacrylat;
Laurylmethacrylat; Vinylidenchlorid; und/oder Tetrafluorethylen.
Als Comonomere B) ebenfalls geeignet sind anorganische Säuren und deren Salze und Ester. Bevorzugte Säuren sind Vinylphosphonsäure, Vinylsulfonsäure, Allylphosphonsäure und Methallylsulfonsäure.

Der Gewichtsanteil der Comonomere B), bezogen auf die Gesamtmasse der Copolymere, kann 0 bis 99,8 Gew.-% betragen und beträgt bevorzugt 0,5 bis 80 Gew.-%, besonders bevorzugt 2 bis 50 Gew.-%.

Als Comonomere C) kommen alle olefinisch ungesättigten Monomere mit kationischer Ladung in Frage, die in der Lage sind, in den gewählten Reaktionsmedien mit Acryloyldimethyltaurinsäure oder deren Salze Copolymere zu bilden. Die dabei resultierende Verteilung der kationischen Ladungen über die Ketten hinweg kann statistisch, alternierend, block- oder gradientenartig sein. Es sei darauf hingewiesen werden, dass unter den kationischen Comonomeren C) auch solche zu verstehen sind, die die kationische Ladung in Form einer betainischen, zwitterionischen, oder amphoteren Struktur tragen.
Comonomere C) im Sinne der Erfindung sind auch aminofunktionalisierte Precursor, die durch polymeranaloge Reaktionen in Ihre entsprechenden quaternären (z.B. Reaktion mit Dimethylsulfat, Methylchlorid), zwitterionischen (z.B. Reaktion mit Wasserstoffperoxid), betainischen (z.B. Reaktion mit Chloressigsäure), oder amphotere Derivate überführt werden können.

Besonders bevorzugt als Comonomere C) sind
Diallyldimethylammoniumchlorid (DADMAC),
[2-(Methacryloyloxy)ethyl]trimethylammoniumchlorid (MAPTAC),
[2-(Acryloyloxy)ethyl]trimethylammoniumchlorid,
[2-Methacrylamidoethyl]trimethylammoniumchlorid,
[2-(Acrylamido)ethyl]trimethylammoniumchlorid,
N-Methyl-2-vinylpyridiniumchlorid
N-Methyl-4-vinylpyridiniumchlorid
Dimethylaminoethylmethacrylat,
Dimethylaminopropylmethacrylamid,
Methacryloylethyl-N-oxid und/oder
Methacryloylethyl-betain.

Der Gewichtsanteil der Comonomeren C) kann, bezogen auf die Gesamtmasse der Copolymere, 0,1 bis 99,8 Gew.-%, besonders bevorzugt 0,5 bis 30 Gew.-% und insbesondere bevorzugt 1 bis 20 Gew.-%, betragen.

Als polymerisationsfähige, siliziumhaltige Komponenten D) sind alle mindestens einfach olefinisch ungesättigten Verbindungen geeignet, die unter den jeweils gewählten Reaktionsbedingungen zur radikalischen Copolymerisation befähigt sind. Dabei muss die Verteilung der einzelnen silikonhaltigen Monomere über die entstehenden Polymerketten hinweg nicht notwendigerweise statistisch erfolgen. Auch die Ausbildung von beispielsweise block- (auch multiblock-) oder gradientenartigen Strukturen ist im Sinne der Erfindung. Kombinationen von zwei oder mehreren unterschiedlichen silikonhaltigen Vertretern sind auch möglich. Die Verwendung von silikonhaltigen Komponenten mit zwei oder mehr polymerisationsaktiven Gruppen führt zum Aufbau verzweigter oder vernetzter Strukturen.

Bevorzugte silikonhaltige Komponenten sind solche gemäß Formel (I).

R¹ - Z- [(Si(R³R⁴)-O-)_{w}-(Si(R⁵R⁶)-O)ₓ-]- R² (I)

Dabei stellt R¹ eine polymerisationsfähige Funktion aus der Gruppe der vinylisch ungesättigten Verbindungen dar, die zum Aufbau polymerer Strukturen auf radikalischem Wege geeignet ist. Bevorzugt stellt R¹ einen Vinyl-, Allyl-, Methallyl-, Methylvinyl-, Acryl- (CH₂=CH-CO-), Methacryl- (CH₂=C[CH₃]-CO-), Crotonyl-, Senecionyl-, ltaconyl-, Maleinyl-, Fumaryl- oder Styrylrest dar. Zur Anbindung der silikonhaltigen Polymerkette an die reaktive Endgruppe R¹ ist eine geeignete chemische Brücke Z erforderlich. Bevorzugte Brücken Z sind -O-, -((C₁ - C₅₀)Alkylen)-, -((C₆ - C₃₀) Arylen)-, -((C₅ - C₈) Cycloalkylen)-, -((C₁-C₅₀)Alkenylen)-, -(Polypropylenoxid)ₙ-, -(Polyethylenoxid)ₒ-, -(Polypropylenoxid)ₙ(Polyethylenoxid)ₒ-, wobei n und o unabhängig voneinander Zahlen von 0 bis 200 bedeuten und die Verteilung der EO/PO-Einheiten statistisch oder blockförmig sein kann. Weiterhin geeignet als Brückegruppierungen Z sind -((C₁ - C₁₀)Alkyl)-(Si(OCH₃)₂)- und -(Si(OCH₃)₂)-.
Der polymere Mittelteil wird durch silikonhaltige Wiederholungseinheiten repräsentiert.
Die Reste R³, R⁴, R⁵ und R⁶ bedeuten unabhängig voneinander -CH₃, -O-CH₃, -C₆H₅ oder -O-C₆H₅.
Die Indizes w und x repräsentieren stöchiometrische Koeffizienten, die unabhängig voneinander 0 bis 500, bevorzugt 10 bis 250, betragen.
Die Verteilung der Wiederholungseinheiten über die Kette hinweg kann nicht nur rein statistisch, sondern auch blockartig, alternierend oder gradientenartig sein kann.
R² kann einerseits einen aliphatischen, olefinischen, cycloaliphatischen, arylaliphatischen oder aromatischen (C₁ - C₅₀)-Kohlenwasserstoffrest symbolisieren (linear oder verzweigt) oder -OH, -NH₂, -N(CH₃)₂, -R⁷ oder für die Struktureinheit [-Z-R¹] stehen. Die Bedeutung der beiden Variablen Z und R¹ wurde bereits erklärt. R⁷ steht für weitere Si-haltige Gruppierungen. Bevorzugte R⁷-Reste sind -O-Si(CH₃)₃, -O-Si(Ph)₃, -O-Si(O-Si(CH₃)₃)₂CH₃) und -O-Si(O-Si(Ph)₃)₂Ph).
Wenn R² ein Element der Gruppe [-Z-R¹] darstellt, handelt es sich um difunktionelle, Monomere, die zur Vernetzung der entstehenden Polymerstrukturen herangezogen werden können.
Formel (I) beschreibt nicht nur vinylisch funktionalisierte, silikonhaltige Polymerspezies mit einer polymertypischen Verteilung, sondern auch definierte Verbindungen mit diskreten Molekulargewichten.

Besonders bevorzugte silikonhaltige Komponenten sind die folgenden acrylisch- oder methacrylisch modifizierten silikonhaltigen Komponenten:

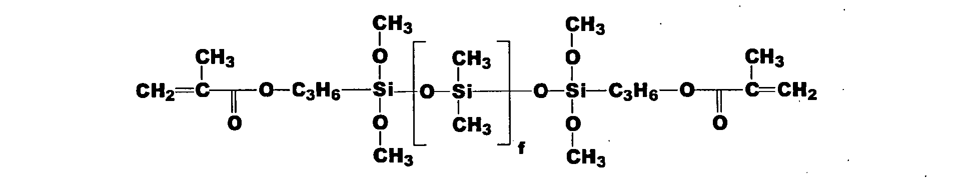

Methacryloxypropyldimethylsilyl endgeblockte Polydimethylsiloxane (f=2 bis 500)

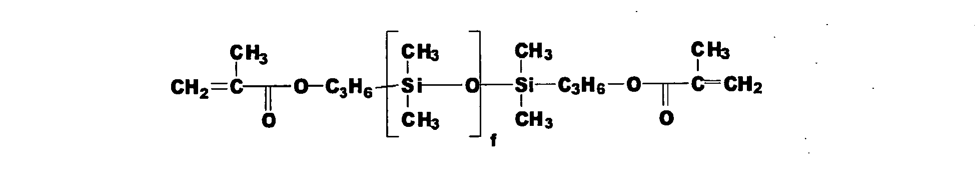

Methacryloxypropyl endgeblockte Polydimethylsiloxane (f= 2 bis 500 bis)

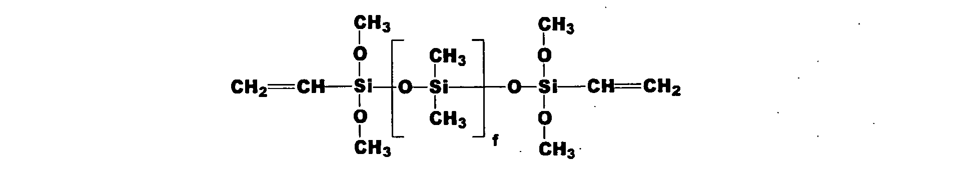

Vinyldimethoxysilyl endgeblockte Polydimethylsiloxane (f=2-500)

Bezogen auf die Gesamtmasse der Copolymere kann der Anteil an siliziumhaltigen Komponenten bis 99,9 Gew.-%, bevorzugt 0,5 bis 30 Gew.-%, insbesondere bevorzugt 1 bis 20 Gew.-%, betragen.

Als polymerisationsfähige, fluorhaltige Komponenten E) sind alle mindestens einfach olefinisch ungesättigten Verbindungen geeignet, die unter den jeweils gewählten Reaktionsbedingungen zur radikalischen Copolymerisation befähigt sind. Dabei muss die Verteilung der einzelnen fluorhaltigen Monomere über die entstehenden Polymerketten hinweg nicht notwendigerweise statistisch erfolgen. Auch die Ausbildung von beispielsweise block- (auch multiblock-) oder gradientenartigen Strukturen ist im Sinne der Erfindung. Kombinationen von zwei oder mehreren unterschiedlichen, fluorhaltigen Komponenten E) ist auch möglich, wobei dem Experten klar ist, dass monofunktionelle Vertreter zur Bildung kammförmiger Strukturen führen, wohingegen di-, tri-, oder polyfunktionelle Komponenten E) zu zumindest teilvernetzten Strukturen führen.
Bevorzugte fluorhaltige Komponenten E) sind solche gemäß Formel (II).

R¹-Y-CᵣH₂ᵣCₛF₂ₛCF₃ (II)

Dabei stellt R¹ eine polymerisationsfähige Funktion aus der Gruppe der vinylisch ungesättigten Verbindungen dar, die zum Aufbau polymerer Strukturen auf radikalischem Wege geeignet ist. Bevorzugt stellt R¹ ein Vinyl-, Allyl-, Methallyl-, Methylvinyl-, Acryl- (CH₂=CH-CO-), Methacryl- (CH₂=C[CH₃]-CO-), Crotonyl-, Senecionyl-, ltaconyl-, Maleinyl-, Fumaryl- oder Styrylrest, besonders bevorzugt einen Acryl- und Methacrylrest, dar.
Zur Anbindung der fluorhaltigen Gruppierung an die reaktive Endgruppe R¹ ist eine geeignete chemische Brücke Y erforderlich. Bevorzugte Brücken Y sind -O-, -C(O)-, -C(O)-O-, -S-, -O-CH₂-CH(O-)-CH₂OH, -O-CH₂-CH(OH)-CH₂-O-, -O-SO₂-O-, -O-S(O)-O-, -PH-, -P(CH₃)-, -PO₃-, -NH-, -N(CH₃)-, -O-(C₁-C₅₀)Alkyl-O-, -O-Phenyl-O-, -O-Benzyl-O-, -O-(C₅-C₈)Cycloalkyl-O-, -O-(C₁-C₅₀)Alkenyl-O-, -O-(CH(CH₃)-CH₂-O)ₙ-, -O-(CH₂-CH₂-O)ₙ- und -O-([CH-CH₂-O]ₙ-[CH₂-CH₂-O]ₘ)ₒ-, wobei n, m und o unabhängig voneinander Zahlen von 0 bis 200 bedeuten und die Verteilung der EO- und PO-Einheiten statistisch oder blockförmig sein kann.
Bei r und s handelt es sich um stöchiometrische Koeffizienten, die unabhängig voneinander Zahlen von 0 bis 200 bedeuten.

Bevorzugte fluorhaltige Komponenten E) gemäß Formel (II) sind
Perfluorhexylethanol-methacrylat,
Perfluorhexoylpropanol-methacrylat,
Perfluoroctyethanol-methacrylat,
Perfluoroctylpropanol-methacrylat,
Perfluorhexylethanolylpolygycolether-methacrylat,
Perfluorhexoyl-propanolyl-poly-[ethylglykol-co-propylenglycolether]-acrylat,
Perfluoroctyethanolyl-poly-[ethylglykol-blockco-propylenglycolether]-methacrylat,
Perfluoroctylpropanolyl-polypropylen-glycolether-methacrylat.

Bezogen auf die Gesamtmasse des Copolymeren kann der Gehalt an fluorhaltigen Komponenten bis 99,9 Gew.-%, bevorzugt 0,5 bis 30 Gew.-%, insbesondere bevorzugt 1 bis 20 Gew.-%, betragen.

Bei den Makromonomeren F) handelt sich um mindestens einfach olefinisch funktionalisierte Polymere mit einer oder mehreren diskreten Wiederholungseinheiten und einem zahlenmittleren Molekulargewicht größer oder gleich 200 g/mol. Bei der Copolymerisation können auch Mischungen chemisch unterschiedlicher Makromonomere F) eingesetzt werden. Bei den Makromonomeren handelt es sich um polymere Strukturen, die aus einer oder mehreren Wiederholungseinheit(en) aufgebaut sind und eine für Polymere charakteristische Molekulargewichtsverteilung aufweisen.

Bevorzugt als Makromonomere F) sind Verbindungen gemäß Formel (III).

R¹-Y-[(A)ᵥ-(B)_{w}-(C)ₓ-(D)_{z}]-R² (III)

R¹ stellt eine polymerisationsfähige Funktion aus der Gruppe der vinylisch ungesättigten Verbindungen dar, die zum Aufbau polymerer Strukturen auf radikalischem Wege geeignet sind. Bevorzugt stellt R¹ einen Vinyl-, Allyl-, Methallyl-, Methylvinyl-, Acryl- (CH₂=CH-CO-), Methacryl- (CH₂=C[CH₃]-CO-), Crotonyl-, Senecionyl-, ltaconyl-, Maleinyl-, Fumaryl- oder Styrylrest dar. Zur Anbindung der Polymerkette an die reaktive Endgruppe ist eine geeignete verbrückende Gruppe Y erforderlich. Bevorzugte Brücken Y sind -O-, -C(O)-, -C(O)-O-, -S-, -O-CH₂-CH(O-)-CH₂OH, -O-CH₂-CH(OH)-CH₂O-, -O-SO₂-O-, -O-SO₂-O-, -O-SO-O-, -PH-, -P(CH₃)-, -PO₃-, -NH- und -N(CH₃)-, besonders bevorzugt -O-.
Der polymere Mittelteil des Makromonomeren wird durch die diskreten Wiederholungseinheiten A, B, C und D repräsentiert. Bevorzugte Wiederholungseinheiten A,B,C und D leiten sich ab von Acrylamid, Methacrylamid, Ethylenoxid, Propylenoxid, AMPS, Acrylsäure, Methacrylsäure, Methylmethacrylat, Acrylnitril, Maleinsäure, Vinylacetat, Styrol, 1,3-Butadien, Isopren, Isobuten, Diethylacrylamid und Diisopropylacrylamid.
Die Indizes v, w, x und z in Formel (III) repräsentieren die stöchiometrische Koeffizienten betreffend die Wiederholungseinheiten A, B, C und D. v, w, x und z betragen unabhängig voneinander 0 bis 500, bevorzugt 1 bis 30, wobei die Summe der vier Koeffizienten im Mittel ≥ 1 sein muss.
Die Verteilung der Wiederholungseinheiten über die Makromonomerkette kann statistisch, blockartig, alternierend oder gradientenartig sein.
R² bedeutet einen linearen oder verzweigten aliphatischen, olefinischen, cycloaliphatischen, arylaliphatischen oder aromatischen (C₁-C₅₀)-Kohlenwasserstoffrest, OH, -NH₂, -N(CH₃)₂ oder ist gleich der Struktureinheit [-Y-R¹].
Im Falle von R² gleich [-Y-R¹] handelt es sich um difunktionelle Makromonomere, die zur Vernetzung der Copolymere geeignet sind.

Besonders bevorzugt als Makromonomere F) sind acrylisch- oder methacrylisch monofunktionalisierte Alkylethoxylate gemäß Formel (IV). 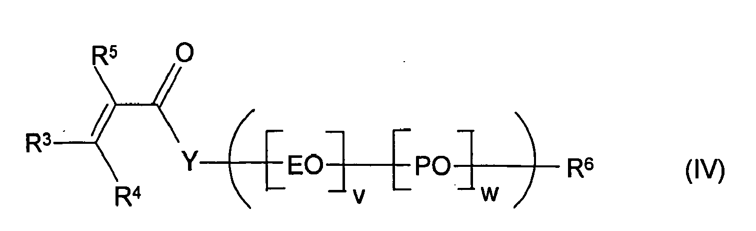 R₃, R₄, R₅ und R₆ bedeuten unabhängig voneinander Wasserstoff oder n-aliphatische, iso-aliphatische, olefinische, cycloaliphatische, arylaliphatische oder aromatische (C₁-C₃₀)-Kohlenwasserstoffreste.
Bevorzugt sind R₃ und R₄ gleich H oder -CH₃, besonders bevorzugt H; R₅ ist gleich H oder -CH₃; und R₆ ist gleich einem n-aliphatischen, iso-aliphatischen, olefinischen, cycloaliphatischen, arylaliphatischen oder aromatischen (C₁-C₃₀)-Kohlenwasserstoffrest.
v und w sind wiederum die stöchiometrischen Koeffizienten betreffend die Ethylenoxideinheiten (EO) und Propylenoxideinheiten (PO). v und w betragen unabhängig voneinander 0 bis 500, bevorzugt 1 bis 30, wobei die Summe aus v und w im Mittel ≥ 1 sein muss. Die Verteilung der EO- und PO-Einheiten über die Makromonomerkette kann statistisch, blockartig, alternierend oder gradientenartig sein. Y steht für die obengenannten Brücken.

Weiterhin insbesondere bevorzugte Makromonomeren F) haben die folgende Struktur gemäß Formel (IV):

| Bezeichnung | R³ | R⁴ | R⁵ | R⁶ | v | w |
|---|---|---|---|---|---|---|
| ^{®}LA-030-methyacrylat | H | H | -CH₃ | -Lauryl | 3 | 0 |
| ^{®}LA-070-methacrylat | H | H | -CH₃ | -Lauryl | 7 | 0 |
| ^{®}LA-200-methacrylat | H | H | -CH₃ | -Lauryl | 20 | 0 |
| ^{®}LA-250-methacrylat | H | H | -CH₃ | -Lauryl | 25 | 0 |
| ^{®}T-080-methyacrylat | H | H | -CH₃ | -Talk | 8 | 0 |
| ^{®}T-080-acrylat | H | H | H | -Talk | 8 | 0 |
| ^{®}T-250-methyacrylat | H | H | -CH₃ | -Talk | 25 | 0 |
| ^{®}T-250-crotonat | -CH₃ | H | -CH₃ | -Talk | 25 | 0 |
| ^{®}OC-030-methacrylat | H | H | -CH₃ | -Octyl | 3 | 0 |
| ^{®}OC-105-methacrylat | H | H | -CH₃ | -Octyl | 10 | 5 |
| ^{®}Behenyl-010-methyaryl | H | H | H | -Behenyl | 10 | 0 |
| ^{®}Behenyl-020-methyaryl | H | H | H | -Behenyl | 20 | 0 |
| ^{®}Behenyl-010-senecionyl | -CH₃ | -CH₃ | H | -Behenyl | 10 | 0 |
| ^{®}EG-440-diacrylat | H | H | H | -Acryl | 10 | 0 |
| ^{®}B-11-50-methacrylat | H | H | -CH₃ | -Butyl | 17 | 13 |
| ^{®}MPEG-750-methacrylat | H | H | -CH₃ | -Methyl | 18 | 0 |
| ^{®}P-010-acrylat | H | H | H | -Phenyl | 10 | 0 |
| ^{®}O-050-acrylat | H | H | H | -Oleyl | 5 | 0 |

Weiterhin als Makromonomere F) insbesondere geeignet sind Ester der (Meth)acrylsäure mit
(C₁₀-C₁₈)-Fettalkoholpolyglykolethern mit 8 EO-Einheiten (Genapol^{®} C-080)
C₁₁-Oxoalkoholpolyglykolethern mit 8 EO-Einheiten (Genapol^{®} UD-080)
(C₁₂-C₁₄)-Fettalkoholpolyglykolethern mit 7 EO-Einheiten (Genapol^{®} LA-070)
(C₁₂-C₁₄)-Fettalkoholpolyglykolethern mit 11 EO-Einheiten (Genapol^{®} LA-110)
(C₁₆-C₁₈)-Fettalkoholpolyglykolethern mit 8 EO-Einheiten (Genapol^{®} T-080)
(C₁₆-C₁₈)-Fettalkoholpolyglykolethern mit 15 EO-Einheiten (Genapol^{®} T-150)
(C₁₆-C₁₈)-Fettalkoholpolyglykolethern mit 11 EO-Einheiten (Genapol^{®} T-110)
(C₁₆-C₁₈)-Fettalkoholpolyglykolethern mit 20 EO-Einheiten (Genapol^{®} T-200)
(C₁₆-C₁₈)-Fettalkoholpolyglycolethern mit 25 EO-Einheiten (Genapol^{®} T-250)
(C₁₈-C₂₂)-Fettalkoholpolyglykolethern mit 25 EO-Einheiten und/oder
iso-(C₁₆-C₁₈)-Fettalkoholpolyglycolethern mit 25 EO-Einheiten
Bei den Genapol^{®}-Typen handelt es sich um Produkte der Firma Clariant, GmbH.

Bevorzugt beträgt das Molekulargewicht der Makromonomeren F) 200 g/mol bis 10⁶ g/mol, besonders bevorzugt 150 bis 10⁴ g/mol und insbesondere bevorzugt 200 bis 5000 g/mol.

Bezogen auf die Gesamtmasse der Copolymere können geeignete Makromonomere bis zu 99.9 Gew.-% eingesetzt werden. Bevorzugt finden die Bereiche 0.5 bis 30 Gew.-% und 70 bis 99,5 Gew.-% Anwendung. Besonders bevorzugt sind Anteile von 1 bis 20 Gew.-% und 75 bis 95 Gew.-%.

Bevorzugt als Copolymere sind solche, die durch Copolymerisation mindestens der Komponenten A), C) und D) erhältlich sind.

Weiterhin bevorzugt als Copolymere sind solche, die durch Copolymerisation mindestens der Komponenten A), C) und E) erhältlich sind.

Weiterhin bevorzugt als Copolymere sind solche, die durch Copolymerisation mindestens der Komponenten A), D) und F) erhältlich sind.

Weiterhin bevorzugt als Copolymere sind solche, die durch Copolymerisation mindestens der Komponenten A) und F) erhältlich sind.

Weiterhin bevorzugt als Copolymere sind solche, die durch Copolymerisation mindestens der Komponenten A) und D) erhältlich sind.

Weiterhin bevorzugt als Copolymere sind solche, die durch Copolymerisation mindestens der Komponenten A) und E) erhältlich sind.

In einer bevorzugten Ausführungsform wird die Copolymerisation in Gegenwart mindestens eines polymeren Additivs G) durchgeführt, wobei das Additiv G) vor der eigentlichen Copolymerisation dem Polymerisationsmedium ganz- oder teilweise gelöst zugegeben wird. Die Verwendung von mehreren Additiven G) ist ebenfalls erfindungsgemäß. Vernetzte Additive G) können ebenfalls verwendet werden.
Die Additive G) bzw. deren Mischungen müssen lediglich ganz oder teilweise im gewählten Polymerisationsmedium löslich sein. Während des eigentlichen Polymerisationsschrittes hat das Additiv G) mehrere Funktionen. Einerseits verhindert es im eigentlichen Polymerisationsschritt die Bildung übervernetzter Polymeranteile im sich bildenden Copolymerisat und andererseits wird das Additiv G) gemäß dem allgemein bekannten Mechanismus der Pfropf-Copolymerisation statistisch von aktiven Radikalen angegriffen. Dies führt dazu, dass je nach Additiv G) mehr oder weniger große Anteile davon in die Copolymere eingebaut werden. Zudem besitzen geeignete Additive G) die Eigenschaft, die Lösungsparameter der sich bildenden Copolymere während der radikalischen Polymerisationsreaktion derart zu verändern, dass die mittleren Molekulargewichte zu höheren Werten verschoben werden. Verglichen mit analogen Copolymeren, die ohne den Zusatz der Additive G) hergestellt wurden, zeigen solche, die unter Zusatz von Additiven G) hergestellt wurden, vorteilhafterweise eine signifikant höhere Viskosität in wässriger Lösung.
Bevorzugt als Additive G) sind in Wasser und/oder Alkoholen, bevorzugt in t-Butanol, lösliche Homo- und Copolymere. Unter Copolymeren sind dabei auch solche mit mehr als zwei verschiedenen Monomertypen zu verstehen. Besonders bevorzugt als Additive G) sind Homo- und Copolymere aus N-Vinylformamid, N-Vinylacetamid, N-Vinylpyrrolidon, Ethylenoxid, Propylenoxid, Acryloyldimethyltaurinsäure, N-Vinylcaprolactam, N-Vinylmethylacetamid, Acrylamid, Acrylsäure, Methacrylsäure, N-Vinylmorpholid, Hydroxyethylmethacrylat, Diallyldimethylammoniumchlorid (DADMAC) und/oder [2-(Methacryloyloxy)ethyl]trimethylammoniumchlorid (MAPTAC); Polyalkylenglykole und/oder Alkylpolyglykole.
Insbesondere bevorzugt als Additive G) sind Polyvinylpyrrolidone (z.B. Luviskol K15^{®}, K20^{®} und K30^{®} von BASF), Poly(N-Vinylformamide), Poly(N-Vinylcaprolactame) und Copolymere aus N-Vinylpyrrolidon, N-Vinylformamid und/oder Acrylsäure, die auch teilweise oder vollständig verseift sein können. Das Molekulargewicht der Additive G) beträgt bevorzugt 10² bis 10⁷ g/mol, besonders bevorzugt 0,5*10⁴ bis 10⁶ g/mol.

Die Einsatzmenge des polymeren Additivs G) beträgt, bezogen auf die Gesamtmasse der bei der Copolymerisation zu polymerisierenden Monomere, bevorzugt 0,1 bis 90 Gew.-%, besonders bevorzugt 1 bis 20 Gew.-% und insbesondere bevorzugt 1,5 bis 10 Gew.-%.

In einer weiteren bevorzugten Ausführungsform sind die erfindungsgemäßen Copolymere vernetzt, d.h. sie enthalten Comonomere mit mindestens zwei polymerisationsfähigen Vinylgruppen.
Bevorzugte Vernetzer sind Methylenbisacrylamid; Methylenbismethacrylamid; Ester ungesättigter Mono- und Polycarbonsäuren mit Polyolen, bevorzugt Diacrylate und Triacrylate bzw. -methacrylate, besonders bevorzugt Butandiol- und Ethylenglykoldiacrylat bzw. -methacrylat, Trimethylolpropantriacrylat (TMPTA) und Trimethylolpropantrimethacrylat (TMPTMA); Allylverbindungen, bevorzugt Allyl(meth)acrylat, Triallylcyanurat, Maleinsäurediallylester, Polyallylester, Tetraallyloxyethan, Triallylamin, Tetraallylethylendiamin; Allylester der Phosphorsäure; und/oder Vinylphosphonsäurederivate.
Insbesondere bevorzugt als Vernetzer ist Trimethylolpropantriacrylat (TMPTA). Der Gewichtsanteil an vernetzenden Comonomeren, bezogen auf die Gesamtmasse der Copolymere, beträgt bevorzugt bis 20 Gew.-%, besonders bevorzugt 0,05 bis 10 Gew.-% und insbesondere bevorzugt 0,1 bis 7 Gew.-%.

Als Polymerisationsmedium können alle organischen oder anorganischen Lösungsmittel dienen, die sich bezüglich radikalischer Polymerisationsreaktionen weitestgehend inert verhalten und vorteilhafterweise die Bildung mittlerer oder hoher Molekulargewichte zulassen. Bevorzugt Verwendung finden Wasser; niedere Alkohole; bevorzugt Methanol, Ethanol, Propanole, iso-, sec.- und t-Butanol, insbesondere bevorzugt t-Butanol; Kohlenwasserstoffe mit 1 bis 30 Kohlenstoffatomen und Mischungen der vorgenannten Verbindungen.

Die Polymerisationsreaktion erfolgt bevorzugt im Temperaturbereich zwischen 0 und 150°C, besonders bevorzugt zwischen 10 und 100°C, sowohl bei Normaldruck als auch unter erhöhtem oder erniedrigtem Druck. Gegebenenfalls kann die Polymerisation auch unter einer Schutzgasatmosphäre, vorzugsweise unter Stickstoff, ausgeführt werden.

Zur Auslösung der Polymerisation können energiereiche elektromagnetische Strahlen, mechanische Energie oder die üblichen chemischen Polymerisationsinitiatoren, wie organische Peroxide, z.B. Benzoylperoxid, tert.-Butylhydroperoxid, Methylethylketonperoxid, Cumolhydroperoxid, Dilauroylperoxid oder Azoinitiatoren, wie z.B. Azodiisobutyronitril (AIBN), verwendet werden. Ebenfalls geeignet sind anorganische Peroxyverbindungen, wie z.B. (NH₄)₂S₂O₈, K₂S₂O₈ oder H₂O₂, gegebenenfalls in Kombination mit Reduktionsmitteln (z.B. Natriumhydrogensulfit, Ascorbinsäure, Eisen(II)-sulfat etc.) oder Redoxsystemen, welche als reduzierende Komponente eine aliphatische oder aromatische Sulfonsäure (z.B. Benzolsulfonsäure, Toluolsulfonsäure etc.) enthalten.

Als Polymerisationsmedium können alle Lösungsmittel dienen, die sich bezüglich radikalischer Polymerisationsreaktionen weitestgehend inert verhalten und die Bildung hoher Molekulargewichte zulassen. Bevorzugt Verwendung finden Wasser und niedere, tertiäre Alkohole oder Kohlenwasserstoffe mit 3 bis 30 C-Atomen. In einer besonders bevorzugten Ausführungsweise wird t-Butanol als Reaktionsmedium verwendet. Mischungen aus zwei- oder mehreren Vertretern der beschriebenen potentiellen Lösungsmitteln sind selbstverständlich ebenfalls erfindungsgemäß. Dies schließt auch Emulsionen von nicht miteinander mischbaren Solventien ein (z.B. Wasser / Kohlenwasserstoffe). Grundsätzlich sind alle Arten der Reaktionsführung geeignet, die zu den erfindungsgemäßen Polymerstrukturen führen (Lösungspolymerisation, Emulsionsverfahren, Fällungsverfahren, Hochdruckverfahren, Suspensionsverfahren, Substanzpolymerisation, Gelpolymerisation usw.).
Bevorzugt eignet sich die Fällungspolymerisation, besonders bevorzugt die Fällungspolymerisation in tert.-Butanol.

Die nachfolgende Auflistung zeigt 67 Copolymere, die für die Formulierung der erfindungsgemäßen Mittel besonders geeignet sind. Die verschiedenen Copolymere Nr. 1 bis Nr. 67 sind gemäß den folgenden Herstellverfahren 1, 2, 3 und 4 erhältlich.

### Verfahren 1:

Diese Polymere sind nach dem Fällungsverfahren in tert. Butanol herstellbar. Dabei wurden die Monomere in t-Butanol vorgelegt, die Reaktionsmischung inertisiert und anschließend die Reaktion nach Anheizen auf 60°C durch Zugabe des entsprechenden t-Butanol löslichen Initiators (bevorzugt Dilauroylperoxid) gestartet. Die Polymere werden nach beendeter Reaktion (2 Stunden) durch Absaugen des Lösungsmittels und durch anschließende Vakuumtrocknung isoliert.

### Verfahren 2:

Diese Polymere sind nach dem Gelpolymerisationsverfahren in Wasser herstellbar. Dabei werden die Monomere in Wasser gelöst, die Reaktionsmischung inertisiert und anschließend die Reaktion nach Anheizen auf 65°C durch Zugabe von geeigneten Initiatoren- oder Initiatorsystemen (bevorzugt Na₂S₂O₈) gestartet. Die Polymergele werden anschließend zerkleinert und nach Trocknung die Polymere isoliert.

### Verfahren 3:

Diese Polymere sind nach dem Emulsionsverfahren in Wasser herstellbar. Dabei werden die Monomere in einer Mischung aus Wasser/organ. Lösungsmittel (bevorzugt Cyclohexan) unter Verwendung eines Emulgators emulgiert, die Reaktionsmischung mittels N₂ inertisiert und anschließend die Reaktion nach Anheizen auf 80°C durch Zugabe von geeigneten Initiatoren- oder Initiatorsystemen (bevorzugt Na₂S₂O₈) gestartet. Die Polymeremulsionen werden anschließend eingedampft (Cyclohexan fungiert als Schlepper für Wasser) und dadurch die Polymere isoliert.

### Verfahren 4:

Diese Polymere sind nach dem Lösungsverfahren in organischen Lösungsmitteln (bevorzugt Toluol, z.B. auch tert. Alkohole) herstellbar. Dabei werden die Monomere im Lösungsmittel vorgelegt, die Reaktionsmischung inertisiert und anschließend die Reaktion nach Anheizen auf 70°C durch Zugabe von geeigneten Initiatoren- oder Initiatorsystemen (bevorzugt Dilauroylperoxid) gestartet. Die Polymere werden durch Abdampfen des Lösungsmittels und durch anschließende Vakuumtrocknung isoliert.

Polymere mit hydrophoben Seitenketten, unvernetzt

| Nr. | Zusammensetzung | Herstellverfahren |
|---|---|---|
| 1 | 95 g AMPS 5 g Genapol T-080 | 1 |
| 2 | 90 g AMPS 10 g Genapol T-080 | 1 |
| 3 | 85 g AMPS 15 g Genapol T-080 | 1 |
| 4 | 80 g AMPS 20 g Genapol T-080 | 1 |
| 5 | 70 g AMPS 30 g Genapol T-080 | 1 |
| 6 | 50 g AMPS 50 g Genapol T-080 | 3 |
| 7 | 40 g AMPS 60 g Genapol T-080 | 3 |
| 8 | 30 g AMPS 70 g Genapol T-080 | 3 |
| 9 | 20 g AMPS 80 g Genapol T-080 | 3 |
| 10 | 60 g AMPS 60 g BB10 | 4 |
| 11 | 80 g AMPS 20 g BB10 | 4 |
| 12 | 90 g AMPS 10 g BB10 | 3 |
| 13 | 80 g AMPS 20 g BB10 | 1 |
| 14 | 80 g AMPS 20 g Genapol LA040 | 1 |

Polymere mit hydrophoben Seitenketten, vernetzt

| Nr. | Zusammensetzung | Herstellverfahren |
|---|---|---|
| 15 | 80 g AMPS 20 g Genapol LA040 0.6g AMA | 1 |
| 16 | 80 g AMPS 20 g Genapol LA040 0,8 AMA | 1 |
| 17 | 80 g AMPS 20 g Genapol LA040 1,0 g AMA | 1 |
| 18 | 628,73 g AMPS 120,45 g Genapol T-250 6,5 g TMPTA | 2 |
| 19 | 60 g AMPS 40 g BB10 1,9 g TMPTA | 4 |
| 20 | 80 g AMPS 20 g BB10 1,4 g TMPTA | 4 |
| 21 | 90 g AMPS 10 g BB10 1,9 g TMPTA | 4 |
| 22 | 80 g AMPS 20 g BB10 1,9 g TMPTA | 4 |
| 23 | 60 g AMPS 40 g BB10 1,4 g TMPTA | 4 |

Polymere mit hydrophoben Seitenketten, vernetzt, gepfropft

| Nr. | Zusammensetzung | Herstellverfahren |
|---|---|---|
| 24 | 95 g AMPS 5 g BB10, 1,9 g TMPTA, 1 g Poly-NVP | 1 |
| 25 | 90 g AMPS 10 g BB10, 1,9 g TMPTA, 1 g Poly-NVP | 1 |
| 26 | 85 g AMPS 15 g BB10, 1,9 g TMPTA, 1 g Poly-NVP | 1 |
| 27 | 90 g AMPS 10 g BB10, 1,9 g TMPTA, 1 g Poly-NVP | 1 |

Polymere mit siliziumhaltigen Gruppen, unvernetzt

| Nr. | Zusammensetzung | Herstellverfahren |
|---|---|---|
| 28 | 80 g AMPS, 20 g Silvet 867 | 1 |
| 29 | 80 g AMPS, 50 g Silvet 867 | 4 |

Polymere mit siliziumhaltigen Gruppen, vernetzt

| Nr. | Zusammensetzung | Herstellverfahren |
|---|---|---|
| 30 | 80 g AMPS, 20 g Silvet 867, 0,5 g MBA | 4 |
| 31 | 80 g AMPS, 20 g Silvet 867, 1,0 g MBA | 1 |
| 32 | 60 g AMPS, 40 g Y-12867, 0,95 g AMA | 1 |
| 33 | 80 g AMPS, 20 g Y-12867, 0,95 g AMA | 1 |
| 34 | 90 g AMPS, 10 g Y-12867, 0,95 g AMA | 1 |
| 35 | 60 g AMPS, 40 g Silvet 7280, 0,95 g AMA | 1 |
| 36 | 80 g AMPS, 20 g Silvet 7280, 0,95 g AMA | 1 |
| 37 | 90 g AMPS, 10 g Silvet 7280, 0,95 g AMA | 1 |
| 38 | 60 g AMPS, 40 g Silvet 7608, 0,95 g AMA | 1 |
| 39 | 80 g AMPS, 20 g Silvet 7608, 0,95 g AMA | 1 |
| 40 | 90 g AMPS, 10 g Silvet 7608, 0,95 g AMA | 1 |

Polymere mit hydrophoben Seitenketten und kationischen Gruppen, unvernetzt

| Nr. | Zusammensetzung | Herstellverfahren |
|---|---|---|
| 41 | 87,5 g AMPS, 7,5 g Genapol T-110, 5 g DADMAC | 2 |
| 42 | 40 g AMPS, 10 g Genapol T110, 45 g Methacrylamid | 2 |
| 43 | 55 g AMPS, 40 g Genapol LA040, 5 g Quat | 1 |
| 44 | 75 g AMPS, 10 g BB10, 6,7 g Quat | 1 |

Polymere mit hydrophoben Seitenketten und kationischen Gruppen, vernetzt

| Nr. | Zusammensetzung | Herstellverfahren |
|---|---|---|
| 45 | 60 g AMPS, 20 g Genapol T-80, 10 g Quat, 10 g HEMA | 1 |
| 46 | 75 g AMPS, 20 g GenapolT-250, 5 g Quat, 1,4 g TMPTA | 1 |
| 47 | 75 g AMPS, 20 g GenapolT-250, 10 g Quat, 1,4 g TMPTA | 1 |
| 48 | 75 g AMPS, 20 g GenapolT-250, 20 g Quat, 1,4 g TMPTA | 1 |

Polymere mit fluorhaltigen Gruppen

| Nr. | Zusammensetzung | Herstellverfahren |
|---|---|---|
| 49 | 94 g AMPS, 2,02 g Fluowet AC 600 | 1 |
| 50 | 80 g AMPS, 20 g Perfluoroctylpolyethylenglykolmethacrylat, 1 g Span 80 | 3 |

Polymere mit fluorhaltigen Gruppen, gepfropft

| Nr. | Zusammensetzung | Herstellverfahren |
|---|---|---|
| 51 | 80 g AMPS, 10 g Fluowet AC 600, 5 g Poly-NVP | 1 |
| 52 | 70 g AMPS, 8 g Perfluoroctylethyloxyglycerinmethacrylat, 5 g Poly-NVP | 4 |

Multifunktionelle Polymere

| Nr. | Zusammensetzung | Herstellverfahren |
|---|---|---|
| 53 | 80 g AMPS, 10 g Genapol LA070, 10 g Silvet 7608, 1,8 g TMPTA | 1 |
| 54 | 70 g AMPS, 5 g N-Vinylpyrrolidon, 15 g Genapol T-250 methacrylat, 10 g Quat, 10 g Poly-NVP | 4 |
| 55 | 80 g AMPS, 5 g N-Vinylformamid, 5 g Genapol O-150-methacrylat, 10 g DADMAC, 1,8 g TMPTA, 8 g Poly-N-Vinylformamid | 2 |
| 56 | 70 g AMPS, 5 g N-Vinylpyrrolidon, 15 g Genapol T-250-methacrylat, 10 g Quat, 10 g Poly-NVP | 1 |
| 57 | 60 g AMPS, 10 g Genapol-BE-020-methacrylat, 10 g Genapol T-250-acrylat, 20 g Quat, 1 g Span 80 | 1 |
| 58 | 60 g AMPS, 20 g MPEG-750-methacrylat, 10 g Methacryloxypyldimethicon, 10 g Perfluorooctylpolyethylenglycol-methacrylat, 10 g Poly[N-vinylcaprolacton-co-acrylsäure] (10/90) | 1 |
| 59 | 80 g AMPS, 5 g N-Vinylformamid, 5 g Genapol O-150-methacrylat, 10 g DADMAC, 1,8gTMPTA | 1 |
| 60 | 70 g AMPS, 10 g Genapol T-250-acrylat, 5 g N-Methyl-4-vinylpyridiniumchlorid, 2,5 g Silvet Y-12867, 2,5 g Perfluorhexylpolyethylenglykolmethacrylat, 10 g Polyethylenglykoldimethacrylat, 4 g Poly[N-Vinylcaprolactam] | 1 |
| 61 | 10 g AMPS, 20 g Acrylamid, 30 g N-2-Vinylpyrrolidon, 20 g Silvet 7608, 10 g Methacryloxypyl dimethicon, 10 g Fluowet AC 812 | 3 |
| 62 | 60 g AMPS, 10 g DADMAC, 10 g Quat, 10 g Genapol-LA-250-crotonat, 10 g Methacryloxypyldimethicon, 7 g Poly[acrylsäure-co-N-vinylformamid] | 1 |
| 63 | 50 g AMPS, 45 g Silvet 7608, 1,8 g TMPTA, 8 g Poly[N-Vinylformamid] | 1 |
| 64 | 20 g AMPS, 10 g Genapol T 110, 35 g MAA, 30 g HEMA, 5 g DADMAC | 4 |
| 65 | 20gAMPS, 80gBB10, 1,4gTMPTA | 1 |
| 66 | 75 g AMPS, 20 g BB10, 6,7 g Quat, 1,4 g TMPTA | 1 |
| 67 | 35 g AMPS, 60 g Acrylamid, 2 g VIFA, 2,5 g Vinylphosphonsäure, 2 Mol-% Fluowet EA-600 | 4 |

Chemische Bezeichnung der Reaktanden:
- AMPS: Acryloyldimethyltaurat, wahlweise Na- oder NH4-Salz
- Genapol^{®} T-080: C₁₆-C₁₈-Fettalkoholpolyglykolether mit 8 EO-Einheiten
- Genapol^{®} T-110: C₁₂-C₁₄-Fettalkoholpolyglykolether mit 11 EO-Einheiten
- Genapol^{®} T-250: C₁₆-C₁₈-Fettalkoholpolyglycolether mit 25 EO-Einheiten
- Genapol^{®} LA-040: C₁₂-C₁₄-Fettalkoholpolyglykolether mit 4 EO-Einheiten
- Genapol^{®} LA-070: C₁₂-C₁₄-Fettalkoholpolyglykolether mit 7 EO-Einheiten
- Genapol^{®} O-150 methacrylat: C₁₆-C₁₈-Fettalkoholpolyglykolether methacrylat mit 15 EO-Einheiten,
- Genapol^{®} LA-250 crotonat: C₁₂-C₁₄-Fettalkoholpolyglykolether crotonat mit 25 EO-Einheiten
- Genapol^{®} T-250 methacrylat: C₁₆-C₁₈-Fettalkoholpolyglycolether methacrylat mit 25 EO-Einheiten
- Genapol^{®} T-250 acrylat: C₁₆-C₁₈-Fettalkoholpolyglycolether methacrylat mit 25 EO-Einheiten
- BB10^{®}: Polyoxyethylen(10)Behenylether
- TMPTA: Trimethylolpropantriacrylat
- Poly-NVP: Poly-N-Vinylpyrrolidon
- Silvet^{®} 867: Siloxan Polyalkylenoxid Copolymer
- MBA: Methylen-bis-acrylamid
- AMA: Allylmethacrylat
- ^{®}Y-12867: Siloxan Polyalkylenoxid Copolymer
- Silvet^{®} 7608: Polyalkylenoxid-modifiziertes
- Heptamethyltrisiloxan Silvet^{®} 7280: Polyalkylenoxid-modifiziertes
- Heptamethyltrisiloxan DADMAC: Diallyldimethyl-ammoniumchlorid
- HEMA: 2-Hydroxyethylmethacrylat
- Quat: 2-(Methacryloyloxy)ethyltrimethylammoniumchlorid
- Fluowet^{®} AC 600: Perfluoralkylethylacrylat
- Span^{®} 80: Sorbitanester

Die beschriebene, optional durchführbare Pfropfung der Copolymere mit anderen Polymeren führt zu Produkten mit besonderer Polymermorphologie, die in wässrigen Systemen optisch klare Gele ergeben. Ein potenzieller Nachteil der Copolymere ohne Pfropfung besteht in einer mehr oder weniger starken Opaleszenz in wässriger Lösung. Diese beruht auf bisher nicht zu vermeidenden, übervernetzten Polymeranteilen, die während der Synthese entstehen und in Wasser nur unzureichend gequollen vorliegen. Dadurch bilden sich Licht streuende Teilchen aus, deren Größe deutlich oberhalb der Wellenlänge des sichtbaren Lichts liegt und deshalb Ursache der Opaleszenz sind. Durch das beschriebene, optional durchführbare Pfropfverfahren wird die Bildung übervernetzter Polymeranteile gegenüber herkömmlichen Techniken deutlich reduziert oder gänzlich vermieden.

Die beschriebene, optional durchführbare Inkorporation sowohl von kationischen Ladungen als auch von Silizium-, Fluor oder Phosphoratomen in die Copolymere führt zu Produkten, die in kosmetischen Formulierungen besondere sensorische und rheologische Eigenschaften besitzen. Eine Verbesserung der sensorischen und rheologischen Eigenschaften kann insbesondere bei der Verwendung in Rinse-off Produkten vorteilhaft sein.

Siliziummodifizierte Copolymere können die Funktionen von Silikonölen in teilweise oder in vollem Umfang übernehmen. Der Einsatz von Silikonen kann durch die Copolymere reduziert oder vermieden werden.

Vorteilhafte Eigenschaften zeigen die Copolymere sowohl in vernetzter, insbesondere aber in unvernetzter Form. Während vernetzte Systeme z.B. hervorragende Eigenschaftsprofile im Hinblick auf Emulsionsstabilisierung zeigen, können insbesondere mit Hilfe der unvernetzten Varianten tensidhaltige Lösungen verdickt werden. Gleiches gilt für elektrolythaltige Systeme, die bekanntermaßen mit Polyelektrolyten nur sehr schwer oder gar nicht zu verdicken sind. Die verdickende Wirkung der Copolymere in wässrig-tensidischen Mitteln wird durch eine Assoziation der Polymerseitenketten und der Tenside verstärkt und kann durch die Wahl der Seitenketten der Polymere und durch die Wahl der Tenside gesteuert werden.

**Tabelle 1: Verdickungsleistung von Copolymer in 15 %iger Tensidlösung in Abhängigkeit vom Verhältnis Genapol LRO zu Genagen CAB**

| | | | | | | | |
|---|---|---|---|---|---|---|---|
| LRO/CAB 02:08 | 03:07 | 04:06 | 05:05 | 06:04 | 07:03 | 08:02 | 05:05 |
| Polymer 0,50 | 0,50 | 0,50 | 0,50 | 0,50 | 0,50 | 0,50 | 0,00 |
| Spindel 3 | 7 | 7 | 7 | 7 | 7 | 2 | 2 |
| Viskosität 4000 [mPas] | 56000 | 53000 | 50000 | 50000 | 56200 | 600 | 90 |
| Viskositätsmessung: | | RV-Brookfield Viskosimeter, 20 U/min, 20°C | | | | | |
| Copolymer: | | 80 g AMPS + 20 g Genapol LA 070 | | | | | |
| ^{®}Genapol LRO: | | (Clariant GmbH, Sodium Laureth Sulfate) | | | | | |
| ^{®}Genagen CAB | | (Clariant GmbH Cocamidopropyl Betaine) | | | | | |
| Gewichtsanteil Polymer: | | 0,5 % | | | | | |
| Gewichtsanteil Tensid: | | 15 % | | | | | |
| E-Wasser: | | ad 100 % | | | | | |
| Temp.: | | 25°C | | | | | |

Das erfindungsgemäße Polymer verdickt signifikant wässrige Tensidsysteme. Die Verdickungsleistung ist abhängig vom Tensidsystem. Man erhält transparente Gele.

**Tabelle 2: Schaumstabilisierende Wirkung von Copolymer-Tensidlösung**

| | 1% Tensid | |
|---|---|---|
| | 30 sec. | 5 min. |
| Genapol LRO | 240 | 210 |
| Genagen CAB | 240 | 210 |
| Hostapon CCG | 290 | 280 |
| Genaminox CSL | 270 | 250 |
| Genapol LRO : Genagen CAB 7:3 | 260 | 240 |
| | | |
| | 1 % Tensid + 0,03 % Copolymer | |
| Genapol LRO | 245 | 230 |
| Genagen CAB | 240 | 225 |
| Hostapon CCG | 290 | 285 |
| Genaminox CSL | 280 | 260 |
| Genapol LRO : Genagen CAB 7:3 | 260 | 255 |

Es wurde die Schaumhöhe und die Schaumstabilität von verschiedenen Tensiden bei Zugabe von 3 % Copolymer (bezogen auf 100% Tensid) nach Ross-Miles untersucht. Hierzu wurden 1%ige Tensid- Lösungen verschiedener Tenside mit und ohne Zusatz von 0,03 % Copolymer angesetzt.
Prüfbedingungen: 0°dH; pH=7
Copolymer Nr. 41: 87,5 g AMPS, 7,5 g Genapol T-110, 5 g DADMAC
Genapol LRO (CLARIANT, Sodium Laureth Sulfate)
Genagen CAB (CLARIANT Cocamidopropyl Betaine)
Hostapon CCG (CLARIANT. Sodium Cocoyl Glutamate)
Genaminox CSL (CLARIANT, Cocamine Oxide)
Genapol LRO : Genagen CAB 7:3

### Ergebnis:

Der Zusatz von 3% Copolymer ( bezogen auf 100% Tensid) verbessert die Schaumstabilität, gemessen in 1 %igen Tensidlösungen nach 5 min. In einigen Fällen wird auch eine Erhöhung des Initialschaums erzielt.
Außerdem wurde eine Verbesserung in der Schaumstruktur beobachtet. Der Schaum mit dem Zusatz an Polymer ist feinporiger und kompakter.

Die suspendierende bzw. dispergierende und stabilisierende Wirkung der Copolymere in wässrig-tensidischen Mitteln wird durch die Assoziation der Polymerseitenketten bzw. funktionellen Gruppen in Haupt- und Seitenkette und der in wässrig-tensidischen Mitteln unlöslichen flüssigen Komponenten, beispielsweise Silikonöle bzw. der unlöslichen Komponenten, beispielsweise Zink-Pyrethione, bedingt.
Ein weiterer Vorteil der Copolymere ist ihre Multifunktionalität. So können Copolymere aus AMPS und quaternären und/ oder siliziumhaltigen und/oder fluorhaltigen Monomereinheiten neben einem guten Verdickungsvermögen eine konditionierende und pflegende Wirkung für Haut und Haare haben.

Die erfindungsgemäßen Mittel enthalten, bezogen auf die fertigen Mittel, bevorzugt 0,01 bis 10 Gew.-%, vorzugsweise 0,1 bis 5 Gew.-%, insbesondere bevorzugt 0,5 bis 3 Gew.-%, an Copolymeren.
Die erfindungsgemäßen Mittel können als Tenside anionische, kationische, nichtionische, zwitter-ionische und/oder amphotere Tenside enthalten.

Die Gesamtmenge der eingesetzten Tenside beträgt, bezogen auf das fertige Mittel, bevorzugt zwischen 2 bis 70 Gew.-%, besonders bevorzugt zwischen 5 und 40 Gew.-%, insbesondere bevorzugt zwischen 12 und 35 Gew.-%.

Als anionische Tenside eignen sich bevorzugt (C₁₀-C₂₀)-Alkyl- und Alkylencarboxylate, Alkylethercarboxylate, Fettalkoholsulfate, Fettalkoholethersulfate, Alkylamidsulfate und -sulfonate, Fettsäurealkylamidpolyglykolethersulfate, Alkansulfonate und Hydroxyalkansulfonate, Olefinsulfonate, Acylester von Isethionaten, α-Sulfofettsäureester, Alkylbenzolsulfonate,
Alkylphenolglykolethersulfonate, Sulfosuccinate, Sulfobernsteinsäurehalbester und -diester, Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate, Sulforicinoleate, Acylglutamate.
Die Verbindungen und deren Mischungen werden in Form ihrer wasserlöslichen oder in Wasser dispergierbaren Salze benutzt, beispielsweise der Natrium-, Kalium-, Magnesium-, Ammonium-, Mono-, Di- und Triethanolammonium- sowie analogen Alkylammonium-Salze.

Der Gewichtsanteil der anionischen Tenside liegt, bezogen auf das fertige Mittel, bevorzugt im Bereich von 2 bis 30 Gew.-%, besonders bevorzugt 5 bis 25 Gew.-%, insbesondere bevorzugt 12 bis 22 Gew.-%, bezogen auf das fertige Mittel.

Geeignete kationische Tenside sind beispielsweise quartäre Ammoniumsalze wie Di-(C₁₀-C₂₄)-Alkyl-dimethylammoniumchlorid oder -bromid, vorzugsweise Di-(C₁₂-C₁₈)-Alkyl-dimethylammoniumchlorid oder -bromid; (C₁₀-C₂₄)-Alkyl-dimethylethylammoniumchlorid oder -bromid; (C₁₀-C₂₄)-Alkyl-trimethylammoniumchlorid oder -bromid, vorzugsweise Cetyltrimethylammoniumchlorid oder -bromid und (C₂₀-C₂₂)-Alkyl-trimethylammoniumchlorid oder -bromid; (C₁₀-C₂₄)-Alkyldimethylbenzyl-ammoniumchlorid oder -bromid, vorzugsweise (C₁₂-C₁₈)-Alkyldimethylbenzyl-ammoniumchlorid; N-(C₁₀-C₁₈)-Alkyl-pyridiniumchlorid oder -bromid, vorzugsweise N-(C₁₂-C₁₆)-Alkyl-pyridiniumchlorid oder -bromid; N-(C₁₀-C₁₈)-Alkyl-isochinolinium-chlorid, -bromid oder -monoalkylsulfat; N-(C₁₂-C₁₈)-Alkylpolyoylaminoformylmethyl-pyridiniumchlorid; N-(C₁₂-C₁₈)-Alkyl-N-methylmorpholinium-chlorid, -bromid oder -monoalkylsulfat; N-(C₁₂-C₁₈)-Alkyl-N-ethylmorpholinium-chlorid, -bromid oder -monoalkylsulfat; (C₁₆-C₁₈)-Alkyl-pentaoxethylammonium-chlorid; Diisobutyl-phenoxyethoxyethyldimethylbenzylammoniumchlorid; Salze des N,N-Diethylaminoethylstearylamids und -oleylamids mit Salzsäure, Essigsäure, Milchsäure, Zitronensäure, Phosphorsäure; N-Acylaminoethyl-N,N-diethyl-N-methyl-ammoniumchlorid, -bromid oder -monoalkylsulfat und N-Acylaminoethyl-N,N-diethyl-N-benzyl-ammonium-chlorid, -bromid oder - monoalkylsulfat, wobei Acyl vorzugsweise für Stearyl oder Oleyl steht. Der Gewichtsanteil der kationischen Tenside liegt bevorzugt im Bereich von 1 bis 10 Gew.-%, besonders bevorzugt 2 bis 7 Gew.-%, insbesondere besonders bevorzugt 3 bis 5 Gew.-%.

Als nichtionische Tenside eignen sich bevorzugt Fettalkoholethoxylate (Alkylpolyethylenglykole); Alkylphenolpolyethylenglykole; Alkylmercaptanpolyethylenglykole; Fettaminethoxylate (Alkylaminopolyethylenglykole); Fettsäureethoxylate (Acylpolyethylenglykole); Polypropylenglykolethoxylate (Pluronics^{®}); Fettsäurealkylolamide, (Fettsäureamidpolyethylenglykole); N-Alkyl-, N-Alkoxypolyhydroxyfettsäureamid, Saccharoseester; Sorbitester und der Polyglykolether.

Der Gewichtsanteil der nichtionischen Tenside liegt bevorzugt im Bereich von 1 bis 20 Gew.-%, besonders bevorzugt 2 bis 10 %, insbesondere bevorzugt 3 bis 7 Gew.-%.

Bevorzugte Amphotenside sind N-(C₁₂-C₁₈)-Alkyl-β-aminopropionate und N-(C₁₂-C₁₈)-Alkyl-β-iminodipropionate als Alkali- und Mono-, Di- und Trialkylammonium-Salze; N-Acylaminoalkyl-N,N-dimethyl-acetobetain, vorzugsweise N-(C₈-C₁₈)-Acylaminopropyl-N,N-dimethylacetobetain; (C₁₂-C₁₈)-Alkyl-dimethyl-sulfopropyl-betain; Amphotenside auf Basis Imidazolin (Handelsname: Miranol^{®}, Steinapon^{®}, vorzugsweise das Natrium-Salz des 1-(β-Carboxy-methyloxyethyl)-1-(carboxymethyl)-2-lauryl-imidazoliniums; Aminoxid, z.B. (C₁₂-C₁₈)-Alkyldimethylaminoxid, Fettsäureamidoalkyl-dimethylaminoxid.

Der Gewichtsanteil der amphoteren Tenside liegt bevorzugt im Bereich von 0,5 bis 20 Gew.-%, besonders bevorzugt 1 bis 10 Gew.-%.

Besonders bevorzugte Tenside sind Laurylsulfat, Laurethsulfat, Cocoamidopropylbetain, Natriumcocoylglutamat, Lauroamphoacetat

In einer bevorzugten Ausführungsform enthalten die Mittel schaumverstärkende Co-Tenside aus der Gruppe Alkylbetaine, Alkylamidobetaine, Aminopropionate, Aminoglycinate, lmidazoliniumbetaine und Sulfobetaine, Aminoxide und Fettsäurealkanolamide oder Polyhydroxyamide eingesetzt werden.

Die erfindungsgemäßen Mittel können als weitere Hilfs- und Zusatzstoffe Ölkörper, Emulgatoren und Co-Emulgatoren, sowie weitere im kosmetische, pharmazeutische und dermatologischen Bereich gebräuchliche Zusätze, wie z.B. kationische Polymere, Filmbildner, Überfettungsmittel, Stabilisatoren, biogene Wirkstoffe, Glycerin, Konservierungsmittel, Perlglanzmittel, Farb- und Duftstoffe, Lösungsmittel, Trübungsmittel, ferner Eiweißderivate wie Gelatine, Collagenhydrolysate, Polypeptide auf natürlicher und synthetischer Basis, Eigelb, Lecithin, Lanolin und Lanolinderivate, Fettalkohole, Silicone, deodorierende Mittel, Stoffe mit keratolytischer und keratoplastischer Wirkung, Enzyme und Trägersubstanzen enthalten. Des weiteren können den erfindungsgemäßen Mitteln antimikrobiell wirkende Agentien zugesetzt werden.

Unter Ölkörper ist jegliche Fettsubstanz zu verstehen, die bei Raumtemperatur (25°C) flüssig ist.
Die Fett-Phase kann daher ein oder mehrere Öle umfassen, die vorzugsweise aus folgenden Ölen ausgewählt werden:
Silikonöle, flüchtig oder nicht flüchtig, linear, verzweigt oder ringförmig; eventuell organisch modifiziert; Phenylsilikone; Silikonharze und -gummis; Mineralöle wie
Paraffin- oder Vaselinöl; Öle tierischen Ursprungs wie Perhydrosqualen, Lanolin; Öle pflanzlichen Ursprungs wie flüssige Triglyceride, z.B. Sonnenblumen-, Mais-, Soja-, Reis-, Jojoba-, Babusscu-, Kürbis-, Traubenkern-, Sesam-, Walnuss-, Aprikosen-, Makadamia-, Avocado-, Süßmandel-, Wiesenschaumkraut-, Ricinusöl, Triglyceride der Capryl/Caprinsäuren, Olivenöl, Erdnussöl, Rapsöl und Kokosnussöl;
Synthetische Öle wie Purcellinöl, Isoparaffine, lineare und/oder verzweigte Fettalkohole und Fettsäureester, bevorzugt Guerbetalkohole mit 6 bis 18, vorzugsweise 8 bis 10, Kohlenstoffatomen; Ester von linearen (C₆-C₁₃)-Fettsäuren mit linearen (C₆-C₂₀)-Fettalkoholen; Ester von verzweigten (C₆₋C₁₃)-Carbonsäuren mit linearen (C₆-C₂₀)-Fettalkoholen, Ester von linearen (C₆-C₁₈)-Fettsäuren mit verzweigten Alkoholen, insbesondere 2-Ethylhexanol; Ester von linearen und/oder verzweigten Fettsäuren mit mehrwertigen Alkoholen (wie z.B. Dimerdiol oder Trimerdiol) und/oder Guerbetalkoholen; Triglyceride auf Basis (C₆-C₁₀)-Fettsäuren; Ester wie Dioctyladipat, Diisopropyl dimer dilineloat; Propylenglykole/-dicaprilat oder Wachse wie Bienenwachs, Paraffinwachs oder Mikrowachse, gegebenenfalls in Kombination mit hydrophilen Wachsen, wie z.B. Cetylstearylalkohol; Fluorierte und perfluorierte Öle; fluorierte Silikonöle; Gemische der vorgenannten Verbindungen.

Als nichtionogene Co-Emulgatoren kommen u.a. in Betracht Anlagerungsprodukte von 0 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitan- bzw. Sorbitolester; (C₁₂-C₁₈)-Fettsäuremono- und -diester von Anlagerungsprodukten von 0 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und ggfs. deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z.B. Polyglycerinpolyricinoleat und Polyglycerinpoly-12-hydroxystearat. Ebenfalls geeignet sind Gemische von Verbindungen aus mehreren dieser Substanzklassen.

Als ionogene Co-Emulgatoren eignen sich z.B. anionische Emulgatoren, wie mono-, di- oder tri-Phosphorsäureester, aber auch kationische Emulgatoren wie mono-, di- und tri-Alkylquats und deren polymere Derivate.

Als kationische Polymere eignen sich die unter der INCI-Bezeichnung "Polyquaternium" bekannten, insbesondere Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, sowie Polyquaternium 37&mineral oil&PPG trideceth (Salcare SC95), PVPdimethylaminoethylmethacrylat-Copolymer, Guar-hydroxypropyltriammoniumchloride, sowie Calciumalginat und Ammoniumalginat. Des weiteren können eingesetzt werden kationische Cellulosederivate; kationische Stärke; Copolymere von Diallylammoniumsalzen und Acrylamiden; quaternierte Vinylpyrrolidon/ Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z.B. Amidomethicone; Copolymere der Adipinsäure und Dimethylaminohydroxy-propyldiethylentriamin; Polyaminopolyamid und kationische Chitinderivate, wie beispielsweise Chitosan. Geeignete Siliconverbindungen sind beispielsweise Dimethylpolysiloxan, Methylphenylpolysiloxane, cyclische Silicone und amino-, fettsäure-, alkohol-, polyether-, epoyx-, fluor- und/oder alkylmodifizierte Siliconverbindungen, sowie Polyalkylsiloxane, Polyalkylarylsiloxane, Polyethersiloxan-Copolymere, wie in US 5 104 645 und den darin zitierten Schriften beschrieben, die bei Raumtemperatur sowohl flüssig als auch harzförmig vorliegen können.

Geeignete Filmbildner sind, je nach Anwendungszweck Salze der Phenylbenzimidazolsulfonsäure, wasserlösliche Polyurethane, beispielsweise C10-Polycarbamyl Polyglycerolester, Polyvinylalkohol, Polyvinylpyrrolidon, -copolymere, beispielsweise VinylpyrrolidonNinylacetatcopolymer, wasserlösliche Acrylsäurepolymere/ Copolymere bzw. deren Ester oder Salze, beispielsweise Partialestercopolymere der Acryl/ Methacrylsäure und Polyethylenglykolether von Fettalkoholen, wie Acrylat/Steareth-20-Methacrylat Copolymer, wasserlösliche Cellulose, beispielsweise Hydroxymethylcellulose, Hydroxyethylcellulose, Hydroxypropylcellulose, wasserlösliche Quaterniums, Polyquaterniums, Carbocyvinyl-Polymere, wie Carbomere und deren Salze, Polysaccharide, beispielsweise Polydextrose und Glucan.

Als Überfettungsmittel können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Als feuchtigkeitsspendende Substanz stehen beispielsweise Isopropylpalmitat, Glycerin und/ oder Sorbitol zu Verfügung.

Als Stabilisatoren können Metallsalze von Fettsäuren, wie z.B. Magnesium-, Aluminium- und/oder Zinkstearat eingesetzt werden.
Unter biogenen Wirkstoffen sind beispielsweise Pflanzenextrakte und Vitaminkomplexe zu verstehen.

Zusätzlich können die erfindungsgemäßen Mittel organische Lösungsmittel enthalten. Prinzipiell kommen als organische Lösungsmittel alle ein- oder mehrwertigen Alkohole in Betracht. Bevorzugt werden Alkohole mit 1 bis 4 Kohlenstoffatomen wie Ethanol, Propanol, Isopropanol, n-Butanol, i-Butanol, t-Butanol, Glycerin und Mischungen aus den genannten Alkoholen eingesetzt. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und in Mengen bis zu 45 Gew.-% und von Polyethylenglykol mit einer relativen Molekülmasse zwischen 400 und 600 in Mengen von 5 bis 25 Gew.-% bevorzugt. Weitere geeignete Lösungsmittel sind beispielsweise Triacetin (Glycerintriacetat) und 1-Methoxy-2-propanol. Hydrotrop wirken kurzkettige Aniontenside, insbesondere Arylsulfonate, beispielsweise Cumol- oder Toluolsulfonat.

Die erfindungsgemäßen Mittel können mit konventionellen Ceramiden, Pseudoceramiden, Fettsäure-N-alkylpolyhydroxyalkylamiden, Cholesterin, Cholesterinfettsäureestern, Fettsäuren, Triglyceriden, Cerebrosiden, Phospholipiden und ähnlichen Stoffen als Pflegezusatz abgemischt werden.

Als Konservierungsmittel in Betracht kommen beispielsweise Phenoxyethanol, Parabene, Pentandiol oder Sorbinsäure.

Als Farbstoffe können die für kosmetische Zwecke geeigneten und zugelassenen Substanzen verwendet werden.
Als antifungizide Wirkstoffe eignen sich bevorzugt Ketoconazol, Oxiconazol, Terbinafin, Bifonazole, Butoconazole, Cloconazole, Clotrimazole, Econazole, Enilconazole, Fenticonazole, Isoconazole, Miconazole, Sulconazole, Tioconazole Fluconazole, Itraconazole, Terconazole, Naftifine und Terbinafine, Zn-Pyrethion und Oczopyrox.

Bei den erfindungsgemäßen Mitteln handelt es sich bevorzugt um Rinse-off-Formulierungen, insbesondere bevorzugt um Shampoos, Duschbäder, Duschgels, und Schaumbäder. Moderne Rinse-off Produkte haben häufig einen hohen Anteil an konditionierenden Wirkstoffen, die auch aus Ölanteilen bestehen können - folglich können diese Mittel als Emulsionen vorliegen.

Die nachfolgenden Beispiele sollen den Gegenstand der Erfindung näher erläutern, ohne ihn darauf einzuschränken (bei den Prozentangaben handelt es sich um Gew.-%). Bei den in den Beispielen verwendeten Copolymeren handelt es sich um Vertreter der in der Beschreibung bereits aufgeführten besonders bevorzugten Copolymere Nr.1 bis Nr.67. Die Herstellung erfolgte nach den dort angegebenen Verfahren 1, 2, 3 oder 4 unter Verwendung der bevorzugten Initiatoren und Lösemittel.

Beispiele für Tensid- Formulierungen

### Beispiel 1: Duschbad

### Zusammensetzung

| | | |
|---|---|---|
| A | ^{®}GENAPOL LRO liquid (Clariant) Sodium Laureth sulfate | 40,00 % |
| B | Fragrance | 0,30 % |
| C | Wasser | 52,70 % |
| | Dyestuff | q.s. |
| | Preservative | q.s. |
| | ^{®}GENAGEN LDA (Clariant) | 6,00 % |
| | Disodium Lauroamphodiacetate Citric Acid | q.s. |
| D | Copolymer Nr. 12 | 1,00 % |

### Herstellung

| | |
|---|---|
| I | B in A einrühren |
| II | Komponenten aus C nacheinander zu I zugeben |
| III | pH auf 5,5 einstellen |
| IV | Einstellen der Viskosität durch Einrühren von D in II |

### Beispiel 2: Baby Shampoo

### Zusammensetzung

| | | |
|---|---|---|
| A | Wasser | 60,70 % |
| | ^{®}GENAPOL ZRO liquid (Clariant) Sodium Laureth Sulfate | 25,00 % |
| | ^{®}HOSTAPON CLG (Clariant) Sodium Lauroyl Glutamate | 8,00 % |
| | ^{®}GENAPOL SBE (Clariant) Disodium Laureth Sulfosuccinate | 5,00 % |
| | Fragrance | 0,30 % |
| | Dyestuff solution | q.s. |
| | Preservative | q.s. |
| B | Copolymer Nr. 26 | 1,00 % |

### Herstellung

| | |
|---|---|
| I | B in A lösen |
| II | gegebenenfalls pH einstellen |

### Beispiel 3: Antischuppen Shampoo, klar

### Zusammensetzung

| | | |
|---|---|---|
| A | ^{®}OCTOPIROX (Clariant) Piroctone Olamine | 0,50 % |
| B | Wasser | 10,00 % |
| C | ^{®}GENAPOL LRO LIQUID (Clariant) Sodium Laureth Sulfate | 30,00 % |
| D | ^{®}Belsil DMC 6032 (Wacker Chemie) Dimethicone Copolyol Acetate | 1,50 % |
| | Fragrance | 0,30 % |
| E | ^{®}ALLANTOIN (Clariant) | 0,30 % |
| F | Wasser | 46,40 % |
| G | Dyestuff solution | q.s. |
| | Panthenol (Hoffmann La Roche) | 1,00 % |
| | ^{®}GENAGEN CAB (Clariant) | 8,00 % |
| | Cocamidopropyl Betaine | |
| H | Copolymer Nr. 49 | 1,10 % |

### Herstellung

| | |
|---|---|
| I | A mit B mischen |
| II | C in I einrühren bis klare Lösung |
| III | Komponenten aus D nacheinander in I geben |
| IV | E in F unter Erwärmen einrühren und dann in I einrühren |
| V | Komponenten aus G nacheinander in I geben |
| VI | gegebenenfalls pH einstellen |
| VII | Einstellen der Viskosität durch Einrühren von H in I |

### Beispiel 4: Antischuppen Shampoo, perlglänzend

### Zusammensetzung

| | | |
|---|---|---|
| A | Wasser | 38,7 % |
| B | ^{®}HOSTAPON SCI-65 (Clariant) Sodium Cocoyl Isethionate | 3,00 % |
| C | ^{®}GENAPOL LRO liquid (Clariant) Sodium Laureth Sulfate | 35,00 % |
| | ^{®}HOSTAPON KCG (Clariant) Sodium Cocoyl Glucamate | 5,00 % |
| | ^{®}Belsil DMC 6032 (Wacker) Dimethicone Copolyol Acetate | 1,00 % |
| | Fragrance | 0,30 % |
| | ^{®}GENAGEN CAB (Clariant) ^{®}Cocamidopropyl Betaine | 9,00 % |
| | GENAPOL TSM (Clariant) PEG-3 Distearate (and) Sodium Laureth Sulfate | 4,00 % |
| | Merquat 550 | 0,50 % |
| | Polyquaternium-7 | |
| | Zinc Omadine FPS (Olin) | |
| | Zinc Pyrithione (48 %ig) | 2,50 % |
| | Copolymer Nr. 2 | 1,00 % |
| | Dyestuff solution | q.s. |
| | Preservative | q.s. |

### Herstellung

| | |
|---|---|
| I | Bei 80°C B in A lösen |
| II | Nach Abkühlen auf ca. 35°C, Komponenten C nacheinander zugeben |

### Beispiel 5: Conditionershampoo

| | | |
|---|---|---|
| I) | Copolymer Nr. 41 | 0,3 % |
| II) | Wasser | 40,0 % |
| III) | Genapol LRO fl. | 39,0 % |
| | Genagen CAB | 15 % |
| IV) | NaCl | 0,2 % |

Zur Herstellung des Conditionershampoos werden I) in II) unter Rühren aufgequollen. Danach werden die Komponenten von III) nacheinander zugegeben. Abschließend wird die Viskosität mit IV) eingestellt. Danach wird der pH-Wert mittels Zitronensäure auf ca. pH=5 eingestellt.

## Patentansprüche

1. Tensidhaltige kosmetische, dermatologische und pharmazeutische Mittel, **dadurch gekennzeichnet, dass** sie mindestens ein wasserlösliches oder wasserquellbares Copolymer, erhältlich durch radikalische Copolymerisation von
A) Acryloyldimethyltaurinsäure und/oder Acryloyldimethyltauraten,
B) gegebenenfalls einem oder mehreren weiteren olefinisch ungesättigten, nicht kationischen, gegebenenfalls vernetzenden, Comonomeren, die wenigstens ein Sauerstoff-, Stickstoff-, Schwefel- oder Phosphoratom aufweisen und ein Molekulargewicht kleiner 500 g/mol besitzen,
C) gegebenenfalls einem oder mehreren olefinisch ungesättigten, kationischen Comonomeren, die wenigstens ein Sauerstoff-, Stickstoff-, Schwefel- oder Phosphoratom aufweisen und ein Molekulargewicht kleiner 500 g/mol besitzen,
D) gegebenenfalls einer oder mehreren mindestens monofunktionellen, zur radikalischen Polymerisation befähigten, siliziumhaltigen Komponente(n),
E) gegebenenfalls einer oder mehreren mindestens monofunktionellen, zur radikalischen Polymerisation befähigten, fluorhaltigen Komponente(n),
F) einem oder mehreren einfach oder mehrfach olefinisch ungesättigten, gegebenenfalls vernetzenden, Makromonomeren, die jeweils mindestens ein Sauerstoff-, Stickstoff-, Schwefel- oder Phosphoratom besitzen und ein zahlenmittleres Molekulargewicht größer oder gleich 200 g/mol aufweisen, wobei es sich bei den Makromonomeren nicht um eine siliziumhaltige Komponente D) oder fluorhaltige Komponente E) handelt,
G) wobei die Copolymerisation gegebenenfalls in Gegenwart mindestens eines polymeren Additivs mit zahlenmittleren Molekulargewichten von 200 g/mol bis 10⁹ g/mol erfolgt,
enthalten.

2. Mittel nach Anspruch 1, **dadurch gekennzeichnet, dass** es sich bei den Comonomeren B) um ungesättigte Carbonsäuren, Salze ungesättigter Carbonsäuren, Anhydride ungesättigter Carbonsäuren, Ester ungesättigter Carbonsäuren mit aliphatischen, olefinischen, cycloaliphatischen, arylaliphatischen oder aromatischen Alkoholen mit 1 bis 22 C-Atomen, offenkettige N-Vinylamide, cyclische N-Vinylamide mit einer Ringgröße von 3 bis 9, Amide der Acrylsäure, Amide der Methacrylsäure, Amide substituierter Acrylsäuren, Amide substituierter Methacrylsäuren, 2-Vinylpyridin, 4-Vinylpyridin, Vinylacetat; Styrol, Acrylnitril, Vinylchlorid, Vinylidenchlorid, Tetrafluorethylen, Vinylphosphonsäure oder deren Ester oder Salze, Vinylsulfonsäure oder deren Ester oder Salze, Allylphosphonsäure oder deren Ester oder Salze und/oder Methallylsulfonsäure oder deren Ester oder Salze handelt.

3. Mittel nach Anspruch 1 und/oder 2, **dadurch gekennzeichnet, dass** es sich beiden Comonomeren C) um
Diallyldimethylammoniumchlorid (DADMAC),
[2-(Methacryloyloxy)ethyl]trimethylammoniumchlorid (MAPTAC),
[2-(Acryloyloxy)ethyl]trimethylammoniumchlorid,
[2-Methacrylamidoethyl]trimethylammoniumchlorid,
[2-(Acrylamido)ethyl]trimethylammoniumchlorid,
N-Methyl-2-vinylpyridiniumchlorid
N-Methyl-4-vinylpyridiniumchlorid
Dimethylaminoethylmethacrylat,
Dimethylaminopropylmethacrylamid,
Methacryloylethyl-N-oxid und/oder
Methacryloylethyl-betain handelt.

4. Mittel nach mindestens einem der Ansprüche 1 bis 3, **dadurch gekennzeichnet, dass** es sich bei den siliziumhaltigen Komponenten D) um Verbindungen der Formel (I)
R¹-Z-[(Si(R³R⁴)-O-)_{w}(Si(R⁵R⁶)-O)ₓ-]-R² (I)
handelt, wobei
R¹ einen Vinyl-, Allyl-, Methallyl-, Methylvinyl-, Acryl-, Methacryl-, Crotonyl-, Senecionyl-, ltaconyl-, Maleinyl-, Fumaryl- oder ein Styrylrest darstellt;
Z eine chemische Brücke, bevorzugt ausgewählt aus -O-, -((C₁ - C₅₀) Alkylen)-,
-((C₆ - C₃₀)-Arylen)-, -((C₅ - C₈) Cycloalkylen)-, -((C₁-C₅₀) Alkenylen)-, -(Polypropylenoxid)ₙ-, -(Polyethylenoxid)ₒ-,
-(Polypropylenoxid)ₙ(Polyethylenoxid)ₒ-, wobei n und o unabhängig voneinander Zahlen von 0 bis 200 bedeuten und die Verteilung der EO/PO-Einheiten statistisch oder blockförmig sein kann;
-((C₁ - C₁₀) Alkyl)-(Si(OCH₃)₂)- und -(Si(OCH₃)₂)-, darstellt;
R³, R⁴, R⁵ und R⁶ unabhängig voneinander -CH₃, -O-CH₃, -C₆H₅ oder -O-C₆H₅ bedeuten;
w, x Zahlen von 0 bis 500 bedeuten, wobei entweder w oder x größer Null sein muss, und
R² einen gesättigten oder ungesättigten, aliphatischen cycloaliphatischen, arylaliphatischen oder aromatischen Rest mit jeweils 1 bis 50 C-Atomen oder eine Gruppe der Formeln -OH, -NH₂, -N(CH₃)₂, -R⁷ oder eine Gruppe -Z-R¹ bedeutet, wobei Z und R¹ die obengenannten Bedeutungen haben und
R⁷ eine Gruppe der Formel -O-Si(CH₃)₃, -O-Si(Phenyl)₃,
-O-Si(O-Si(CH₃)₃)₂CH₃) und -O-Si(O-Si(Ph)₃)₂Ph) bedeutet.

5. Mittel nach mindestens einem der Ansprüche 1 bis 4, **dadurch gekennzeichnet, dass** es sich bei den fluorhaltigen Komponenten E) um Verbindungen der Formel (II)
R¹-Y-CᵣH₂ᵣCₛF₂ₛCF₃ (II)
handelt, wobei
R¹ eine polymerisationsfähige Funktion aus der Gruppe der vinylisch ungesättigten Verbindungen, bevorzugt einen Vinyl-, Allyl-, Methallyl-, Methylvinyl-, Acryl-, Methacryl-, Crotonyl-, Senecionyl-, Itaconyl-, Maleinyl-, Fumaryl- oder Styrylrest, darstellt;
Y eine chemische Brücke, bevorzugt ausgewählt aus -O-, -C(O)-, -C(O)-O-, -S-, -O-CH₂-CH(O-)-CH₂OH, -O-CH₂-CH(OH)-CH₂-O-, -O-SO₂-O-, -O-S(O)-O-, -PH-, -P(CH₃)-, -PO₃-, -NH-, -N(CH₃)-, -O-(C₁-C₅₀)Alkyl-O-, -O-Phenyl-O-, -O-Benzyl-O-, -O-(C₅-C₈)Cycloalkyl-O-, -O-(C₁-C₅₀)Alkenyl-O-, -O-(CH(CH₃)-CH₂-O)ₙ-, -O-(CH₂-CH₂-O)ₙ- und -O-([CH-CH₂-O]ₙ-[CH₂-CH₂-O]ₘ)ₒ-, wobei n, m und o unabhängig voneinander Zahlen von 0 bis 200 bedeuten, darstellt und
r,s stöchiometrische Koeffizienten darstellen, die unabhängig voneinander Zahlen zwischen 0 und 200 sind.

6. Mittel nach mindestens einem der Ansprüche 1 bis 5, **dadurch gekennzeichnet, dass** es sich bei den Makromonomeren F) um Verbindungen der Formel (III) handelt,
R¹-Y-[(A)ᵥ-(B)_{w}-(C)ₓ-(D)_{z}]-R² (III)
wobei R¹ eine polymerisationsfähige Funktion aus der Gruppe der vinylisch ungesättigten Verbindungen, bevorzugt einen Vinyl-, Allyl-, Methallyl-, Methylvinyl-, Acryl-, Methacryl-, Crotonyl-, Senecionyl-, ltaconyl-, Maleinyl-, Fumaryl- oder Styrylrest, darstellt;
Y eine verbrückende Gruppe, bevorzugt -O-, -S-, -C(O)-, -C(O)-O-,
-O-CH₂-CH(O-)-CH₂OH, -O-CH₂-CH(OH)-CH₂O-, -O-SO₂-O-, -O-SO₂-O-,
-O-SO-O-, -PH-, -P(CH₃)-, -PO₃-, -NH- und -N(CH₃)- darstellt;
A, B, C und D unabhängig voneinander diskrete chemische Wiederholungseinheiten, bevorzugt hervorgegangen aus Acrylamid, Methacrylamid, Ethylenoxid, Propylenoxid, AMPS, Acrylsäure, Methacrylsäure, Methylmethacrylat, Acrylnitril, Maleinsäure, Vinylacetat, Styrol, 1,3-Butadien, Isopren, Isobuten, Diethylacrylamid und Diisopropylacrylamid, insbesondere bevorzugt Ethylenoxid, Propylenoxid darstellen;
v, w, x und z unabhängig voneinander 0 bis 500, bevorzugt 1 bis 30, betragen, wobei die Summe aus v, w, x und z im Mittel ≥ 1 ist; und
R² einen linearen oder verzweigten aliphatischen, olefinischen, cycloaliphatischen, arylaliphatischen oder aromatischen (C₁-C₅₀)-Kohlenwasserstoffrest, OH, -NH₂ oder -N(CH₃)₂ darstellt oder gleich [-Y-R¹] ist.

7. Mittel nach mindestens einem der Ansprüche 1 bis 6, **dadurch gekennzeichnet, dass** es sich bei den polymeren Additiven G) um Homo- oder Copolymere aus N-Vinylformamid, N-Vinytäcetamid, N-Vinylpyrrolidon, Ethylenoxid, Propylenoxid, Acryloyldimethyltaurinsäure, N-Vinylcaprolactam, N-Vinylmethylacetamid, Acrylamid, Acrylsäure, Methacrylsäure, N-Vinylmorpholid, Hydroxymethylmethacrylat, Diallyldimethylammoniumchlorid (DADMAC) und/oder [2-(Methacryloyloxy)ethyl]trimethylammoniumchlorid (MAPTAC); Polyalkylenglykole und/oder Alkylpolyglykole handelt.

8. Mittel nach mindestens einem der Ansprüche 1 bis 7, **dadurch gekennzeichnet, dass** die Copolymerisation in Gegenwart mindestens eines polymeren Additivs G) erfolgt.

9. Mittel nach mindestens einem der Ansprüche 1 bis 8, **dadurch gekennzeichnet, dass** die Copolymere vernetzt sind.

10. Mittel nach mindestens einem der Ansprüche 1 bis 9, **dadurch gekennzeichnet, dass** die Copolymere durch Fällungspolymerisätion in tert.-Butanol hergestellt werden.

11. Mittel nach mindestens einem der Ansprüche 1 bis 10, **dadurch gekennzeichnet, dass** sie, bezogen auf die fertigen Mittel, 0,01 bis 10 Gew.-% der Copolymere enthalten.

12. Mittel nach mindestens einem der Ansprüche 1 bis 11; **dadurch gekennzeichnet, dass** sie, bezogen auf die fertigen Mittel, 2 bis 70 Gew.-%, bevorzugt 5 bis 40 Gew.-%, Tenside enthalten.

13. Mittel nach mindestens einem der Ansprüche 1 bis 12, **dadurch gekennzeichnet, dass** sie 2 bis 30 Gew.-%, bevorzugt 5 bis 25 Gew.-%, anionische Tenside enthalten.

14. Mittel nach mindestens einem der Ansprüche 1 bis 13, **dadurch gekennzeichnet, dass** sie 1 bis 10 Gew.-%, bevorzugt 2 bis 7 Gew.-%, kationische Tenside enthalten.

15. Mittel nach mindestens einem der Ansprüche 1 bis 14, **dadurch gekennzeichnet, dass** sie 1 bis 20 Gew.-%, bevorzugt 2 bis 10 %, nichtionische Tenside enthalten.

16. Mittel nach mindestens einem der Ansprüche 1 bis 15, **dadurch gekennzeichnet, dass** sie 0,5 bis 20 Gew.-%, bevorzugt 1 bis 10 Gew.-%, amphotere Tenside enthalten.

17. Mittel nach mindestens einem der Ansprüche 1 bis 16, **dadurch gekennzeichnet, dass** sie als anionische Tenside (C₁₀-C₂₀)-Alkyl- und Alkylencarboxylate, Alkylethercarboxylate, Fettalkoholsulfate, Fettalkoholethersulfate, Alkylamidsulfate und -sulfonate, Fettsäurealkylamidpolyglykolethersulfate, Alkansulfonate und Hydroxyalkansulfonate, Olefinsulfonate, Acylester von Isethionaten, α-Sulfofettsäureester, Alkylbenzolsulfonate, Alkylphenolglykolethersulfonate, Sulfosuccinate, Sulfobernsteinsäurehalbester und -diester, Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate, Sulforicinoleate und/oder Acylglutamate enthalten.

18. Mittel nach mindestens einem der Ansprüche 1 bis 17, **dadurch gekennzeichnet, dass** sie als kationische Tenside quartäre Ammoniumsalze enthalten.

19. Mittel nach mindestens einem der Ansprüche 1 bis 18, **dadurch gekennzeichnet, dass** sie als nichtionische Tenside Fettalkoholethoxylate (Alkylpolyethylenglykole); Alkylphenolpolyethylenglykole; Alkylmercaptanpolyethylenglykole; Fettaminethoxylate (Alkylaminopolyethylenglykole); Fettsäureethoxylate (Acylpolyethylenglykole); Polypropylenglykolethoxylate (Pluronics^{®}); Fettsäurealkylolamide, (Fettsäureamidpolyethylenglykole); N-Alkyl-, N-Alkoxypolyhydroxyfettsäureamid, Saccharoseester; Sorbitester und/oder Polyglykolether enthalten.

20. Mittel nach mindestens einem der Ansprüche 1 bis 19, **dadurch gekennzeichnet, dass** sie als Amphotenside sind N-(C₁₂-C₁₈)-Alkyl-β-aminopropionate, N-(C₁₂-C₁₈)-Alkyl-β-iminodipropionate; N-Acylaminoalkyl-N,N-dimethylacetobetain, (C₁₂-C₁₈)-Alkyl-dimethylsulfopropy-betain; Amphotenside auf Basis Imidazolin und/oder Aminoxide enthalten.

21. Mittel nach mindestens einem der Ansprüche 1 bis 20, **dadurch gekennzeichnet, dass** sie schaumverstärkende Co-Tenside enthalten.

22. Mittel nach mindestens einem der Ansprüche 1 bis 21, **dadurch gekennzeichnet, dass** es sich dabei um Rinse-off-Formulierungen handelt.

23. Mittel nach mindestens einem der Ansprüche 1 bis 22, **dadurch gekennzeichnet, dass** es sich dabei um Shampoos, Duschbäder, Duschgels und Schaumbäder handelt.
